# DEMANDE DE BREVET EUROPEEN

(11) **EP 4 851 285 A1**
(43) Date de publication de la demande: **09.12.2026**
(21) Numéro de dépôt: 25181330.9
(22) Date de dépôt: 06.06.2025
(51) Int. Cl.: C12N 15/10, C12Q 1/6806

(54) **COMPLEXE ET PROCÉDÉ DE PRODUCTION, PROCÉDÉ DE DISTRIBUTION DANS UN CONTENANT ET PROCÉDÉ D'IMMOBILISATION DE PARTICULES D EXTRACTION DE BIOMOLÉCULES DANS UN CONTENANT**

(71) Demandeur: bioMérieux, 69280 MARCY-L'ETOILE (FR)
(72) Inventeur: LAURENT, Alain, 38000 GRENOBLE (FR); GAILLARD, Marie, 38000 GRENOBLE (FR); GEISLER, Océane, 38690 LE GRAND LEMPS (FR)
(74) Mandataire: bioMérieux PI Groupement mandataires

(57) **Abrégé**

L'invention porte sur un complexe destiné à revêtir un support constitué au moins d'une matrice et de particules d'extraction destinées à capturer les acides nucléiques d'un échantillon à tester, ledit complexe étant configuré pour se présenter sous au moins deux états distincts :
- un état solide où la matrice est solide et les particules d'extraction de biomolécules sont incluses et non-mobiles dans la matrice, et
- un état liquide où la matrice est liquide ou solubilisée et les particules d'extraction sont libres de leurs mouvements dans ladite matrice.

L'invention porte également sur un procédé de fabrication d'un tel complexe, d'un procédé de distribution d'un tel complexe dans un récipient ou sur un support ainsi qu'un procédé d'extraction et/ou de détection des acides nucléiques d'un échantillon à tester à l'aide dudit complexe.

## Description

### DOMAINE TECHNIQUE DE L'INVENTION

L'invention concerne le domaine technique de la biologie moléculaire et plus particulièrement des systèmes d'extraction de biomolécules à l'aide de particules d'extraction à partir d'échantillons contenant au moins un analyte. Bien évidemment, la présente invention peut s'appliquer à d'autres domaines techniques auxquels elle serait adaptée.

### ARRIERE-PLAN TECHNOLOGIQUE DE L'INVENTION

Dans les systèmes d'extraction, on réalise une lyse des cellules de l'échantillon pour en extraire ce qu'elles contiennent, en particulier les biomolécules (des acides nucléiques - ADN et/ou ARN- ou des protéines). Ensuite, une capture desdites biomolécules est effectuée pour réaliser des tests d'analyse ou diagnostics.

La lyse des cellules de l'échantillon peut être mécanique, chimique ou une combinaison des deux.

Pour la capture des biomolécules (acides nucléiques et/ou protéines), on utilise la plupart du temps un autre support mécanique que celui utilisé pour la lyse et préférentiellement un autre type de particules. Par exemple, pour capturer les acides nucléiques, on utilise des particules recouvertes de silice. De préférence, ces particules sont magnétiques et sont manipulables via un dispositif aimanté. Ces particules d'extraction magnétiques comprenant de la silice sont aussi communément appelées billes de silice, par exemple NucliSENS^{®} easyMAG^{®} Magnetic Silica (MagSIL - bioMérieux, France). Pour la capture des protéines, on peut utiliser plusieurs techniques (électrophorèse, chromatographie, précipitation). Par exemple, on peut utiliser un support portant un anticorps qui va se lier spécifiquement à la protéine d'intérêt. Il pourrait s'agir de particules magnétiques, avec ou sans silice, portant un anticorps ou une molécule permettant de se lier à la protéine d'intérêt.

Les particules d'extraction liées à des constituants des cellules lysées doivent généralement être lavées. Le lavage des particules a lieu de différentes manières : passage par plusieurs contenants remplis de solutions de lavage identiques ou différentes avec ou sans agitation, par un jeu d'aimantation discontinue dans un même contenant ou par agitation lorsque les particules sont magnétiques.

Ensuite, les biomolécules ou molécules d'intérêt (acides nucléiques et/ou protéines en fonction des caractéristiques du support de capture utilisé et des besoins du test) sont dissociées du support de capture ou particules d'extraction via une étape d'élution à la fin de laquelle on récupère les biomolécules (acides nucléiques et/ou protéines) provenant de l'échantillon à tester, qu'elles soient dans les cellules ou les organites de l'échantillon ou dans la phase liquide de l'échantillon, circulantes.

Enfin, après la phase d'élution, une phase d'amplification des acides nucléiques est effectuée généralement afin d'obtenir une quantité suffisante d'acides nucléiques pour permettre de les détecter ensuite de façon rapide, sensible et fiable.

Lors de ce processus d'extraction des biomolécules de l'échantillon à tester, il apparait évident que l'étape de capture est déterminante pour la suite du processus et en particulier que les particules de capture elles-mêmes sont les éléments clés et cruciaux pour une capture efficace des acides nucléiques et/ou des protéines, pour les performances maximales d'une plateforme ou kit d'extraction et plus globalement, in fine, pour rendre un diagnostic fiable.

Il est donc indispensable que ces particules d'extraction :
- soient en quantité suffisante pour extraire toutes les biomolécules d'intérêt de l'échantillon à tester,
- soient dans les conditions optimales pour se lier aux biomolécules,
- soient toutes extraites, collectées, récupérées à l'issue du processus d'extraction. En effet, une fois ces particules d'extraction collectées, une élution des biomolécules qui y sont liées sera effectuée puis une phase d'amplification aura lieu lorsque les biomolécules sont des acides nucléiques. Les biomolécules capturées seront ensuite détectées en vue de rendre un résultat de diagnostic. Il est essentiel de collecter toutes les particules d'extraction ou un maximum pour ne pas perdre des informations clés apportées par les biomolécules qu'elles capturent et ainsi pouvoir rendre un résultat de test de diagnostic le plus fiable, le plus précis, complet et le plus rapidement possible.

Plusieurs étapes délicates se présentent avec ces particules d'extraction:
- Elles doivent être dispensées dans un récipient dans lequel l'échantillon a été introduit ou sera introduit. Ce récipient est généralement de petite taille (de l'ordre de quelques ml, généralement environ de 2 à 3 ml) et la quantité de particules d'extraction par récipient est d'environ 1 mg ce qui représente un volume de ces particules d'environ une dizaine ou une centaine de µl en fonction de la dilution de ces particules. Il peut être difficile de distribuer une si petite quantité et un si petit volume de particules d'extraction dans le récipient. Ceci requiert beaucoup de précision, d'autant plus que ces particules sédimentent généralement et qu'il faut un système de mélange des particules pour les remettre en suspension. Il est donc difficile d'être précis et reproductible dans la distribution des particules dans le récipient destiné à faire le test. Ce récipient peut être appelé par la suite indifféremment dispositif d'extraction, cartouche d'extraction, barrette d'extraction ou plaque d'extraction. Cette distribution des particules d'extraction se fait généralement lors de l'industrialisation des dispositifs ou cartouches d'extraction qui seront mis en vente. Il est intéressant qu'il y puisse y avoir une automatisation du remplissage des récipients d'extraction des biomolécules. Cependant, dans certains cas, la distribution peut se faire extemporanément ou directement dans l'instrument d'analyse et cette étape est réalisée par le client/laboratoire qui va réaliser le test ou analyse. Il faut donc avoir un processus très reproductible, ce qui n'est pas évident et n'est pas toujours optimal d'un point de vue temps jusqu'à présent.

- Pour dispenser ces particules d'extraction de façon la plus précise possible, une solution serait de les diluer mais leur stabilité pourrait être altérée lorsque le volume de dilution est important. De plus, le volume ou taille du récipient/dispositif les accueillant est un facteur limitant pour le volume de particules diluées à introduire car ce récipient doit pouvoir contenir au moins l'échantillon à tester, le tampon de lyse, les particules d'extraction, un dispositif d'agitation et un dispositif de prélèvement configuré pour récupérer les particules chargées en biomolécules. Pour éviter un débordement du matériel contenu dans le récipient lors du processus de test de l'échantillon mais aussi pour éviter de rendre les particules d'extraction instables, il est donc nécessaire de trouver un système permettant d'éviter d'avoir à diluer les particules d'extraction pour les dispenser.
- Lors du transport du récipient ou cartouche d'extraction contenant les réactifs dont les particules d'extraction, il est très probable que le récipient soit remué, secoué, agité, retourné ou ne reste pas à dans le même plan pendant tout le transport, idéalement à l'horizontale (le plan parallèle à celui du couvercle, du foil ou opercule ou de l'ouverture du récipient). Ceci se produit avant que l'échantillon ne soit ajouté dans le récipient, et dans ce cas, une partie des particules d'extraction va recouvrir les parois du récipient et/ou va être projetée sur les parois et/ou sur le couvercle, le film de scellage du récipient ou l'opercule/foil (en plastique ou aluminium). Ceci est très problématique car non seulement une partie des particules risque d'être perdue lors du retrait du film ou de l'opercule diminuant ainsi la capacité de capture des biomolécules mais ceci provoque également la dissémination de particules d'extraction dans l'instrument d'analyse ou du poste d'analyse si l'analyse est réalisée manuellement, et donc sa contamination et/ou sa salissure.
- De plus, une partie des particules d'extraction peut être projetée sur les parois du récipient d'extraction ou du puits de la cartouche d'extraction dans lesquelles elles sont contenues et le volume d'échantillon et de réactif peut ne pas être suffisant pour que les particules d'extraction présentes sur les parois soient ramenées dans le mélange ou soient submergées par l'échantillon et le tampon de lyse et puissent donc servir à la capture des biomolécules de l'échantillon à tester. Ceci réduit encore les capacités de collecte de ces biomolécules et nuit également à la fiabilité mais aussi à la reproductibilité du test. Il est donc nécessaire de les immobiliser et les isoler ou maintenir ensemble dans le fond du récipient pour éviter ce problème. Ce problème peut être traité par une barrière physique appliquée sur les particules comme un film plastique plaqué au-dessus des particules ou une enveloppe contenant des particules (WO 2021/1 16553 A1) ou l'utilisation d'aimant (WO19057345 A1) pour le maintien des particules en place mais cette solution est, d'une part, difficile à mettre en œuvre et automatisable, et, d'autre part, n'est pas totalement satisfaisante car le film ou l'enveloppe ne sont pas totalement hermétiques laissant échapper des particules d'extraction, ne conservent pas les performances optimales des particules car il y a un séchage de ces dernières ou que le matériau utilisé interagit avec elles ou les autres composants utilisés pour le test biologique ou encore que l'aimant nuit ensuite à la bonne réalisation du test biologique.
- Une fois que la phase de lyse de l'échantillon est effectuée et que les biomolécules sont liées aux particules d'extraction, il est nécessaire de collecter les particules pour effectuer des lavages et ainsi obtenir des biomolécules propres, purifiées, sans agent pouvant nuire à leur détection et dans le cas des acides nucléiques comme biomolécules, il est nécessaire de retirer au maximum des agents pouvant inhiber leur amplification. La collecte des particules d'extraction est une phase délicate mais capitale. Il faut collecter toutes les particules pour avoir le meilleur rendement de capture des biomolécules et un résultat de diagnostic fiable. Si une partie des particules est éparpillée et/ou projetée sur une paroi, et en particulier si certaines particules d'extraction sont hors du liquide (échantillon + réactifs) il va être très difficile de les collecter pour ensuite effectuer leur lavage avant l'élution des biomolécules liées. En effet, cette phase de collecte des particules se fait soit par pipetage du liquide contenant les particules d'extraction, soit à l'aide d'un dispositif magnétique ou aimant plongé dans le liquide qui va rassembler ou agréger les particules puis d'un pipetage de cet agrégat de particules d'extraction. La collecte des particules en vue de leur lavage peut se faire également à l'aide uniquement d'un dispositif magnétique dans le liquide puis d'un transfert de ce dispositif vers le lieu de lavage.
- Lors des phases de production ou d'industrialisation des récipients ou cartouches d'extraction, en plus des problèmes exposés ci-avant, il est indispensable que les particules d'extraction, en particulier les particules à base de silice ne soient pas séchées. Lorsque les particules de silice sont séchées, elles perdent en performance de capture des biomolécules et en particulier de capture des acides nucléiques. Ceci vaut donc pour la phase d'industrialisation mais également pour le stockage à long terme des récipients de test ou cartouches qui doit se faire sans évaporation, à température ambiante, faute de quoi les performances et fiabilité du test seraient altérées.
- Les conditions de stockage des particules d'extraction sont un point majeur car les performances de ces particules doivent être conservées au cours du temps, de préférence à température ambiante et quel que soit le milieu dans lequel elles se trouvent. En effet, en milieu liquide, de nombreux additifs présents dans une solution de stockage peuvent s'adsorber sur les particules d'extraction lors de leur stockage et peuvent dégrader les capacités de liaison des particules aux biomolécules et donc leurs performances d'extraction altérant ainsi le résultat final du test (cf. WO2019175518A1) Il est donc fondamental que les particules puissent être stables lors d'un stockage de longue durée et conserver leurs performances.

### OBJET DE L'INVENTION

Il apparait donc que de nombreux problèmes restent à résoudre au sujet de ces particules d'extraction, magnétiques ou non, lors du processus d'extraction des biomolécules de l'échantillon (qu'on appelle également «préparation de l'échantillon») et lors du transport des supports ou récipient contenant ces particules d'extraction de biomolécules. Ceci doit être résolu afin que toutes les biomolécules d'intérêt susceptibles d'être contenues dans l'échantillon soient capturées via lesdites particules d'extraction, détectées et analysées rapidement, efficacement, de façon fiable et reproductible et idéalement de façon automatisée.

L'objet de la présente invention est d'améliorer les processus d'industrialisation, de distribution, de stockage et d'utilisation des particules d'extraction contenues dans une cartouche d'extraction, d'améliorer la stabilité des particules d'extraction et de simplifier le processus de production du récipient de test ou la cartouche d'extraction contenant les particules d'extraction par un complexe permettant l'immobilisation ensemble de ces particules d'extraction. Ce complexe permet ainsi une compartimentation ou un confinement de ces particules d'extraction dans une zone précise d'un support ou récipient d'extraction, et le tout en vue d'obtenir une extraction des biomolécules propre, efficace, optimale, fiable, rapide, reproductible et automatisable.

À cet effet, l'invention a pour objet un complexe comprenant au moins :
- une matrice soluble dans un tampon aqueux, dans un tampon de lyse des cellules d'un échantillon à tester et/ou dans l'échantillon liquide à tester,
- des particules d'extraction de biomolécules, de préférence des particules de silice et plus préférentiellement des particules de silice magnétiques,
ledit complexe est configuré pour se présenter sous au moins deux états distincts :
- un état solide où la matrice est solide et les particules d'extraction de biomolécules sont incluses et non-mobiles dans la matrice, et
- un état liquide où la matrice est liquide ou solubilisée et les particules d'extraction sont libres de leurs mouvements dans ladite matrice.

L'invention a également pour objet un procédé de fabrication d'un complexe selon l'invention comprenant les étapes suivantes :
a) Prendre des particules d'extraction de biomolécules d'un échantillon à tester,
b) Lorsqu'il est présent, retirer le liquide dans lequel les particules d'extraction de biomolécules d'un échantillon à tester sont contenues, ledit liquide pouvant être un tampon de stockage, un solvant, un tampon aqueux, de l'eau ou une matrice au moins partiellement solubilisée,
c) Mettre en contact lesdites particules d'extraction de biomolécules avec une matrice telle que définie pour l'invention, à une température à laquelle ladite matrice est liquide, pour former un mélange ou une suspension de particules d'extraction des biomolécules à une concentration donnée dans la matrice liquide,
c') Optionnellement, réitérer l'étape b) et c),
d) Refroidir ou laisser refroidir naturellement le mélange ou suspension en vue d'obtenir le complexe sous forme solide.

L'invention a également pour objet un procédé de distribution sur un support ou dans un récipient du complexe selon l'invention ou obtenu selon le procédé de fabrication selon l'invention, comprenant au moins une des étapes suivantes :
A) Lorsque le complexe est à l'état liquide, le couler, le verser ou le dispenser à l'aide d'un ustensile adapté sur un support ou dans un récipient, de préférence un récipient d'analyse biologique,
B) Refroidir ou laisser refroidir naturellement le complexe distribué de sorte à ce qu'une fois à l'état solide le complexe ait un volume plein, dépourvu de volume intérieur et qu'il se présente sous la forme d'un culot,
   ou
C) Lorsque le complexe est sous forme solide, déposer au moins une fraction dudit complexe sur le support ou dans le fond du récipient de sorte qu'il forme un complexe ayant un volume plein, dépourvu de volume intérieur.

L'invention concerne également un procédé d'immobilisation de particules d'extraction de biomolécules à une localisation donnée consistant à mettre en œuvre le complexe, tel que défini dans la présente demande ou obtenu selon le procédé de fabrication ou le procédé de distribution de l'invention, dans un récipient ou sur un support.

L'invention a également pour objet un support ou récipient comprenant un complexe conforme à l'invention ou obtenu comme décrit dans la présente demande, dans lequel le complexe se présente sous la forme d'un volume plein à l'état solide, dépourvu de volume intérieur.

Enfin, l'invention a également pour objet un procédé d'extraction des biomolécules contenues dans l'échantillon à tester comprenant
- Une étape de solubilisation du complexe selon l'invention, du complexe tel qu'obtenu selon le procédé de fabrication de l'invention ou du complexe tel que contenu ou immobilisé sur un support ou dans un récipient selon l'invention, par la mise en contact dudit complexe avec un tampon aqueux, un tampon de lyse et/ou l'échantillon à tester lysé ou non lysé,
- Optionnellement si cette étape n'a pas encore eu lieu, une étape de mise en contact du complexe solubilisé avec l'échantillon à tester lysé ou non lysé,
- Optionnellement si l'échantillon à tester n'a pas déjà été lysé, une étape de lyse de l'échantillon à tester,
- Une étape de capture des biomolécules de l'échantillon à tester par les particules d'extraction libres de leurs mouvements dans de la matrice solubilisée, les biomolécules étant de préférence des acides nucléiques,
- Optionnellement, une étape de lavage des biomolécules capturées par les particules d'extraction,
- Optionnellement, une étape d'élution des biomolécules capturées par les particules d'extraction.

Pour finir, l'invention a également pour objet un procédé de détection des biomolécules contenues dans un échantillon à tester, caractérisé en ce qu'il comprend en outre des étapes du procédé d'extraction décrit précédemment, les étapes suivantes :
- Optionnellement, une étape d'amplification des acides nucléiques lorsque les biomolécules extraites à l'issue du procédé d'extraction des biomolécules décrit ci-dessus sont des acides nucléiques, de préférence l'amplification est réalisée par PCR,
- Une étape de détection des biomolécules par des méthodes appropriées.

Ce complexe et les procédés d'extraction et de détection des biomolécules associés permettent une simplicité de distribution, de manipulation des particules d'extraction, une automatisation de production mais également une plus grande fiabilité, reproductibilité des résultats du test rapidité de test.

### BREVE DESCRIPTION DES FIGURES

[Fig. 1] : Variantes du procédé de solubilisation du complexe de l'invention dans un ou plusieurs récipients pouvant être utilisés pour le procédé d'extraction des biomolécules issues d'un échantillon à tester.
[Fig. 2] : Illustration du problème technique lié aux particules d'extraction qui sont dispersées sur les parois d'un récipient de test.
[Fig. 3] : Schéma du procédé de fabrication du complexe selon l'invention et du procédé de distribution sur un support ou dans un récipient.
[Fig. 4] : Procédé de distribution du complexe selon l'invention et illustration du complexe selon l'invention à l'état liquide, dans lequel la matrice est solubilisée.
[Fig. 5] : Variantes du procédé de distribution du complexe selon l'invention.
[Fig. 6] : Complexe selon l'invention comparé à des solutions déjà existantes.
[Fig. 7] : Complexe selon l'invention sous forme solide dans un récipient.

### DESCRIPTION DETAILLEE DE L'INVENTION

### COMPLEXE ET MATRICE

La présente invention couvre un complexe comprenant au moins :
- une matrice soluble dans un tampon aqueux, dans un tampon de lyse des cellules d'un échantillon à tester et/ou dans l'échantillon liquide à tester,
- des particules d'extraction de biomolécules, de préférence des particules de silice et plus préférentiellement des particules de silice magnétiques,
ledit complexe est configuré pour se présenter sous au moins deux états distincts :
- un état solide où la matrice est solide et les particules d'extraction de biomolécules sont incluses et non-mobiles dans la matrice, et
- un état liquide où la matrice est liquide ou solubilisée et les particules d'extractions sont libres de leurs mouvements dans ladite matrice.

Par complexe, on entend une structure pleine, composée de plusieurs composants de natures différentes. En l'occurrence, le complexe selon l'invention est constitué d'une matrice pouvant se présenter sous plusieurs états (solide, liquide ou solubilisée) et d'éléments solides que sont les particules d'extraction des biomolécules de l'échantillon à tester. Le complexe selon l'invention permet de contenir, d'immobiliser et d'isoler des éléments (au moins les particules d'extraction) en les maintenant à une localisation particulière, choisie. Ce complexe est destiné à revêtir un support ou un récipient pour l'extraction de biomolécules et plus précisément il est destiné à revêtir le fond d'un récipient ou un support, de sorte à former un volume plein, sans volume intérieur ou vide intérieur.

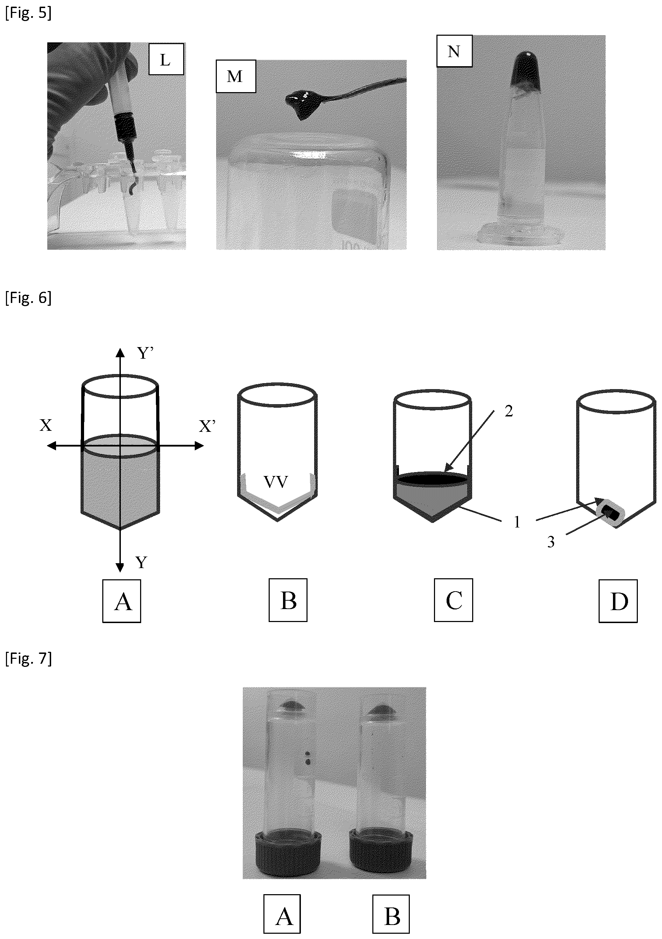

Le volume plein correspond à un volume ayant une hauteur prédéterminée selon un axe longitudinal Y-Y' et une largeur prédéterminée selon un axe transversal X-X' (cf. figure 6) de sorte que le complexe soit continu à la fois le long de l'axe longitudinal mais également le long de l'axe transversal où il forme une surface pleine, continue. Le complexe ne définit pas d'espace ou volume interne. Qu'il soit sous forme solide ou liquide, le complexe selon l'invention remplit au moins le fond du récipient qui le contient en épousant les parois du récipient mais également en comblant l'espace compris entre les parois du récipient, que l'on pourrait appeler l'espace central du récipient. On comprend ainsi que le complexe selon l'invention ne se présente pas sous forme de film ou de membrane qui tapisse uniquement les parois du récipient qui le contient.

Par matrice, on entend une substance inactive qui entoure les particules d'extraction. Dans la présente invention, la matrice est la substance dans laquelle les particules d'extraction sont incluses (lorsqu'elle est à son état solide) ou en suspension (lorsqu'elle est à son état liquide ou solubilisé) mais qui n'a aucune interaction, interférence sur elles.

La matrice est soluble et se trouve à l'état solubilisé lorsqu'elle sera en contact d'une substance aqueuse comme un tampon aqueux, un tampon de lyse des cellules d'un échantillon à tester et/ou d'un échantillon à tester, de préférence un échantillon à l'état liquide. On peut dire également que la matrice est hydrosoluble.

Par hydrosoluble ou soluble dans de l'eau, un tampon aqueux, un tampon de lyse des cellules de l'échantillon à tester et/ou dans l'échantillon liquide à tester, on entend une structure qui se dissout ou se solubilise au moins partiellement dans ces milieux ou environnements, quelle que soit la température du milieu. Ainsi, en conditions aqueuses (eau, tampon aqueux, tampon de lyse de l'échantillon à tester ou échantillon lui-même), cette structure ou substance se dissout dans le milieu dans lequel elle se trouve pour ensemble former une solution ou mélange homogène, uniforme, une suspension colloïdale ou non ou une émulsion. La solubilisation n'est pas nécessairement complète, elle peut n'être que partielle mais elle doit permettre nécessairement au contenu du complexe d'être libéré, mélangé au milieu dans lequel le complexe se trouve de sorte à former une solution, une suspension ou une émulsion. Si la solubilisation n'est que partielle, les portions, fragments non solubilisés ne gênent en rien ou de façon imperceptible, minimale, le procédé d'extraction, de préférence d'extraction des biomolécules, mis en œuvre dans la présente invention. Ils n'interfèrent pas avec un quelconque élément utilisé lors de ce procédé d'extraction, que ce soit chimiquement ou physiquement. Ainsi, si la matrice du complexe n'est pas dissoute totalement, les fragments restants en suspension ne gênent pas physiquement la capture des biomolécules par les particules d'extraction, ni les pipetages nécessaires lors du processus d'extraction des biomolécules ou autres processus physiques ayant lieu au cours du procédé d'extraction ou de détection des biomolécules, de préférence des acides nucléiques. Ils ne gênent pas non plus chimiquement le processus d'extraction des biomolécules. La solution ou suspension créée par la dissolution ou solubilisation de la matrice dans le milieu dans lequel se trouve le complexe n'interfère ou n'altère en rien ou de façon minimale et acceptable la structure des biomolécules (acides nucléiques et/ou protéines) que l'on souhaite capturer et extraire, ni des particules d'extraction qui étaient contenues dans la matrice avant sa dissolution ou solubilisation. Ces dernières conservent toutes leurs performances de capture et d'extraction dans ce nouveau milieu qu'est la suspension ou solution créée à la suite de la dissolution ou la solubilisation, partielle ou totale, de la matrice du complexe.

Il sera compris que par soluble, on entend non seulement qui peut se dissoudre pour former un mélange homogène une fois solubilisé mais on entend également miscible sans créer de démixtion, de mélange biphasique ou de mélange hétérogène. Etant donné que la matrice contient nécessairement des particules d'extraction lorsqu'elle n'est pas solubilisée, il est entendu que lorsqu'on parle de mélange homogène dans sa forme solubilisée, totale ou partielle, on désigne le mélange des liquides extérieurs au complexe ayant permis la solubilisation de la matrice et de la matrice solubilisée.

Il est possible par exemple d'utiliser les paramètres de Hansen pour une matrice choisie où soluble signifie que les paramètres de Hansen se situent à l'intérieur du volume ou de la zone de solubilité pour le ou les composés constituant la matrice, tel que représenté sur une carte de solubilité de Hansen (voir « Hansen Solubility Parameter System » DuPont Nylon Intermediates and Specialties, publication W-400473, 12/2000).

Il existe plusieurs milieux différents pour rendre le complexe à l'état solubilisé et plus précisément la matrice du complexe à l'état solubilisé : de l'eau, un tampon aqueux, un tampon de lyse des cellules d'un échantillon à tester et/ou d'un échantillon à tester, de préférence un échantillon à l'état liquide.

Par tampon aqueux, on entend une solution aqueuse résistant à des changements de pH malgré l'ajout de petites quantités d'acide fort ou de base forte. Ceci permet de maintenir constant le pH de la solution et ainsi ne pas altérer les propriétés de la solution. Ce tampon aqueux peut être par exemple une solution de Tris HCl ou d'acétate de sodium ou du MES, dans des concentrations comprises entre 10 et 100 mM, préférentiellement du Tris HCl à 10 mM et à pH 7 ou une solution d'acétate de sodium à 10 mM à pH 5,5.

Le tampon aqueux selon la présente invention peut contenir un pourcentage défini de co-solvant comme, par exemple, un solvant organique, tel que l'éthanol ou l'isopropanol ou le DMSO. De préférence, le co-solvant est compris dans les proportions (v/v) de 10% ou plus préférentiellement de 5% ou encore plus préférentiellement de 2%.

Le tampon aqueux sera de préférence utilisé avec un volume compris entre 50µl et 3ml. Celui-ci sera adapté en fonction de la taille de l'échantillon, de la taille ou volume du complexe et/ou de l'instrument sur lequel le test sera réalisé. Ceci est bien connu de l'homme du métier qui saura adapter ces choix.

Par tampon de lyse, on entend une solution tamponnée apte à lyser l'échantillon à tester, les cellules de l'échantillon, les organites de l'échantillon, les membranes de l'échantillon. Généralement, le tampon de lyse est un tampon aqueux qui a en outre la fonction de lyse. Le tampon de lyse peut comprendre par exemple un détergent et le cas échéant un agent de chélation. Le pH est généralement maintenu entre 4 et 8, par exemple entre 6 et 8, à l'aide d'un tampon approprié, par exemple le TrisHCl ou l'acétate de sodium ou du MES, éventuellement dans des concentrations comprises entre 10 et 100 mM.

Le tampon de lyse utilisé dans la présente invention peut comprendre des sels par exemple du NaCl, KCl, (NH₄)₂SO₄, de préférence à une concentration comprise entre 0,05M et 0,5M.

Le détergent peut être non-ionique, anionique, cationique ou zwitterionique et peut être choisi parmi les Tween, le Thésit, le Genapol X100, l'Ecosurf, les Tritons, le SDS, les Brij et autres détergents communément utilisés à des concentrations comprises entre 0,05 et 20%.

Eventuellement, le tampon de lyse contient des réactifs pour l'inactivation des nucléases, et/ou l'élimination des protéines, par exemple des protéases telles que la protéinase K. D'autres enzymes peuvent également être utilisées comme les enzymes lytiques (hydrolase, zymolase ...) pour digérer la paroi des levures et des champignons.

Dans un mode de réalisation spécifique, le tampon de lyse ne comprend pas de réactifs pour l'élimination des protéines et notamment, des protéases et ceci spécifiquement lorsque les biomolécules à capturer sont des protéines.

Dans un mode de réalisation particulier, le tampon de lyse comprend également des agents chaotropiques. Les agents chaotropiques interfèrent avec les interactions intramoléculaires faibles (non-covalentes), comme les liaisons hydrogène, les forces de van der Waals et les forces hydrophobes. Parmi les agents chaotropiques, on peut citer l'urée, les sels de guanidine comme le chlorure ou le thiocyanate de guanidinium et le perchlorate de lithium ou de sodium. Ils sont généralement utilisés en concentration allant de 1 à 6 M, en particulier pour le GuSCN et le GuHCl.

Dans un autre mode de réalisation particulier, le tampon de lyse ne comprend pas d'agents chaotropiques.

On peut aussi ajouter des agents de chélation tels que l'EDTA ou des composés similaires, par exemple entre 5 et 50 mM et/ou des composés réducteurs tels que le DTT (dithio-érytréitol) ou du TCEP (Tri carboxy éthyl phosphine) ou du bêta-mercaptoéthanol à des concentrations comprises par exemple entre 0,5 et 100 mM.

Le tampon de lyse peut comprendre en outre un ou des solvants organiques tels que des alcools (éthanol, isopropanol ...) avec un maximum de 30%. Dans un mode de réalisation préféré cependant, le tampon de lyse ne comprend pas de solvants organiques.

Les caractéristiques chimiques d'un échantillon doivent être prises en compte lors de l'optimisation du tampon de lyse. Par exemple, l'acidité d'un échantillon (ex. certains échantillons de sol ou l'urine) peut endommager les acides nucléiques et peut donc être neutralisée pour garantir un rendement correct en acides nucléiques. L'échantillon est mis en contact avec le tampon de lyse pendant un temps suffisant pour permettre la lyse des cellules sans dégrader les acides nucléiques, par exemple entre 0 et 15 min.

Les tampons de lyse suivants sont adaptés pour la présente invention : NUCLISENS^{®} Lysis Buffer de bioMérieux (Référence 200292), Tampon de lyse B - Référence : D2002-1-50 de ZYMO Research, Buffer RLT Plus - Référence : 1053393 de Qiagen, Tampon de lyse PowerLyzer^{®} - Référence : 12855-50 de MOBIO-Qiagen, Tampon de lyse 1X RBC eBioscience^{™} - Référence : 00-4333-57 d'Invitrogen.

Le tampon de lyse peut avoir un volume compris entre 50 µl et 3 ml, de préférence entre 100 µl et 3ml, plus préférentiellement entre 200µl et 2 ml. Le volume du tampon de lyse sera adapté en fonction de la taille de l'échantillon, de la taille ou volume du complexe et/ou de l'instrument sur lequel le test sera réalisé. Ceci est bien connu de l'homme du métier qui saura adapter ces choix.

Dans un mode de réalisation de la présente invention, lorsqu'un tampon de lyse est utilisé, il va agir en lysant les cellules, les membranes de l'échantillon en solubilisant les protéines de l'échantillon tout en évitant la dégradation de celles-ci et leur activité biologique et en régulant le pH et l'osmolarité du lysat. Le tampon de lyse peut non seulement solubiliser la matrice du complexe comprenant les particules d'extraction mais il a également pour but essentiel de rendre accessibles les biomolécules de l'échantillon à analyser, biomolécules qui seront par la suite capturées par les particules d'extraction rendues libres de leurs mouvements grâce à la solubilisation de la matrice.

Les particules d'extraction sont essentielles dans la présente invention. Ce sont des éléments clés pour la capture des biomolécules contenues dans l'échantillon à tester et sont donc essentielles au test lui-même. Elles sont déterminantes pour une capture efficace des biomolécules et pour la fiabilité et la reproductivité du test.

Par particule d'extraction, on entend une structure physique identifiable, distincte, isolée et non soluble dans un milieu aqueux ou un mélange de solvants organiques et aqueux ou dans un solvant organique et dont la taille peut être micrométrique ou nanométrique. De préférence, la particule est nanométrique et entre 1 et 400nm. Cette structure est apte à se lier à des biomolécules tels que des acides nucléiques ou des protéines. De préférence, cette structure est sous forme de particule ou de bille, par exemple des particules de composé(s) magnétique(s) (maghémite, magnétite...) recouvertes de silice car la silice a une affinité très forte pour les acides nucléiques dans des conditions données ou alors elles sont fonctionnalisées pour capturer des protéines. Ces particules sont en général magnétiques grâce à leur composé magnétique, ce qui implique qu'elles sont susceptibles de réagir à un champ magnétique par une réaction d'orientation et/ou de déplacement dépendante de la force et de l'orientation. Cette force s'effectue par l'intermédiaire du champ magnétique, et est produite par des charges en mouvement ou des aimants. Ceci permet de rassembler, collecter et récupérer les particules d'extraction. Les particules d'extraction de l'invention correspondent de préférence à celles décrites dans la demande de brevet WO2016108004A1.

Les particules d'extraction de biomolécules utilisées préférentiellement dans l'invention sont des particules disponibles dans le commerce, telles que les SeraSil-Mag ou les MagPrep^{®} de Merck, les DynaBeads de Thermofisher, les PureSil de BioChain, les NucleoMag de Macherey Nagel, les NucliSENS^{®} easyMAG^{®} Magnetic Silica ou MagSIL - particules de bioMérieux ou tout autre type de particules capables de se lier à et capturer une ou des biomolécules et de préférence les acides nucléiques contenus dans l'échantillon à tester.

Dans le complexe selon l'invention, le volume de particules d'extraction est de préférence d'environ 50µl. Etant donné qu'il ne faut pas que les particules sèchent, ceci correspond au volume des particules d'extraction contenues dans la matrice du complexe. La concentration de particules d'extraction varie entre 5mg/ml à 50mg/ml. Dans une variante préférée de l'invention, le complexe a une concentration de particules d'extraction de 20mg/ml. Ces données sont indicatives et ne sont pas limitatives. Il peut bien évidemment être envisagé des complexes avec des volumes et/ou des concentrations de particules d'extraction différents.

Dans la présente invention, le complexe, lorsqu'il est dans son état solide c'est-à-dire quand la matrice qui le compose est à l'état solide, permet d'immobiliser et d'isoler les particules d'extraction ou de les compartimenter dans un espace défini du récipient ou dispositif d'extraction ou de test. Il permet de former une structure solide qui maintient les particules d'extraction ensemble dans un environnement et les empêche de se disperser dans le récipient dans lequel le complexe est contenu. Ainsi, dans le complexe de l'invention à l'état solide, les particules d'extraction sont incluses dans la matrice et ne peuvent pas bouger, elles y sont figées, immobilisées. Cette matrice à l'état solide remplit donc cette fonction d'immobilisation des particules d'extraction mais également elle les empêche de sécher, ce qui est un double avantage pour le test biologique à réaliser et ce qui résout les problèmes techniques évoqués plus haut. En effet, la nature de la matrice est telle qu'elle ne s'évapore pas, sa solidification se fait grâce à ses propriétés physico-chimiques du ou des composés qui la constituent mais non par évaporation. Ceci est également un avantage dans la mise en œuvre du complexe et son procédé de fabrication qui est plus simple et demande moins d'étapes et de moyens.

Par compartimentation ou immobilisation, on entend la capacité à mettre à l'abri de, à l'écart de, à cloisonner, à confiner, à isoler ou à séparer de. En l'occurrence, le complexe est un moyen pour compartimenter l'espace du récipient de test et permettre d'immobiliser et d'isoler les particules d'extraction dans une localisation définie et de façon rassemblée sans qu'elles puissent se disperser sur les parois du récipient de test et sans qu'elles sèchent ou se dégradent. C'est grâce à la nature et aux propriétés de la matrice du complexe qu'il est possible de produire cette compartimentation ou immobilisation des particules d'extraction. Le complexe dans son état solide, c'est-à-dire lorsque la matrice est sous forme solide, piège les particules d'extraction qui ne sont alors plus libres de leurs mouvements.

Dans un mode de réalisation de l'invention, le complexe, dans son état solide, a une viscosité supérieure ou égale à 10 Pa.s.

Cette viscosité est apportée par la nature de la matrice du complexe. Généralement, la matrice dans son état solide peut présenter un aspect visqueux, pâteux, malléable et/ou sous forme de cristaux ou de gel. Elle permet au complexe d'adhérer aux parois du récipient qui le contient ou au support qui le porte et ceci est intrinsèque à la nature physico-chimique de la matrice. La viscosité de la matrice dans son état solide est mesurée grâce à des techniques connues de l'homme du métier et en particulier exposées dans la littérature (More solutions to sticky problems - A guide to getting more from your Brookfield viscosimeter-Brookfield ; Bases théoriques de la mesure de viscosité - Labomat : https://labomat.eu/fr.faq/586-bases-theoriques-de-la-mesure-de-viscosite.html ; test de pénétration ; BYK guide de la mesure de la viscosité : https://wwww.byk-instruments.com/fr/t/knowledeg/viscometry-measurement; Comment mesurer la viscosité coécrit par Bess Ruff, MA : https.//fr.wikihow.com/mesurer-la-viscosité). De préférence, la viscosité du complexe selon l'invention est mesurée à température ambiante.

Par température ambiante, on entend compris entre 18°C et 25°C, de préférence entre 18°C et 23°C, plus préférentiellement entre 20°C et 22°C.

Dans un mode de réalisation particulier, l'invention concerne un complexe dans lequel la matrice est :
- solide à une température située en-dessous d'une première température déterminée,
- liquide entre cette première température et 200°C, et
- solubilisée lorsqu'elle est en contact d'un tampon aqueux, d'un tampon de lyse des cellules d'un échantillon à tester et/ou d'un échantillon liquide à tester.

Ainsi, la matrice est sous forme solide lorsqu'elle se trouve à une température située en-dessous d'une première température déterminée qui correspond au point de fusion du ou des composés constituant la matrice. Ce point de fusion dépend donc de la nature chimique du ou des composés formant la matrice. Le point de fusion correspond à la température à laquelle une substance passe de l'état solide à l'état liquide.

La matrice est sous forme liquide lorsqu'elle se trouve à une température située entre le point de fusion du ou des composés la constituant et 200°C. La matrice se liquéfie à la chaleur et au-delà de son point de fusion.

Par exemple, si la matrice est constituée de tert-butanol, le point de fusion de ce dernier se trouve à 25°C, la matrice se trouve donc à l'état solide lorsqu'elle est située en dessous d'une première température correspondant au point de fusion du tert-butanol donc en-dessous de 25°C. Elle se trouve à l'état liquide lorsqu'elle est à une température comprise entre 25°C et 200°C. Si la matrice est constituée et 2,2, diméthyl-1-propanol, elle se trouvera à l'état solide en-dessous de 50°C (qui est la température déterminée du point de fusion de ce composé) et à l'état liquide à une température comprise entre 50°C et 200°C. Il sera donc bien compris que l'état solide et liquide de la matrice va dépendre de la nature chimique du ou des composés la constituant. Ainsi, en fonction de ce ou ces constituants de la matrice, l'homme du métier saura déterminer lorsque la matrice sera à l'état solide ou liquide.

Dans un mode particulier de réalisation, l'invention concerne un complexe tel que défini ci-dessus, dans lequel la matrice a un point de fusion compris entre 20°C et 85°C, de préférence entre 22°C et 80°C, plus préférentiellement entre 23°C et 75°C.

Il est bien entendu que la matrice est à l'état liquide à une température située au-dessus de son point de fusion et jusqu'à 200°C. Au-delà de 200°C, les fonctionnalités et performances des particules d'extraction ainsi que les propriétés physico-chimiques des constituants de la matrice risquent d'être endommagés, il est donc recommandé de ne pas chauffer le complexe selon l'invention au-delà de cette température. Ainsi, dans un mode particulier de l'invention, la matrice du complexe est sous forme liquide à une température comprise entre son point de fusion (qui équivaut au point de fusion du ou des composés la constituant) et 180°C, plus préférentiellement entre son point de fusion et 150°C, encore plus préférentiellement entre le point de fusion et 130°C.

Il sera compris que la matrice est un constituant essentiel du complexe selon l'invention et c'est sa nature particulière permettant une forme solide et une forme liquide ou solubilisée qui participe à la résolution des problèmes techniques évoqués plus haut.

Dans un mode de réalisation spécifique, l'invention concerne un complexe tel que défini ci-dessus, dans lequel la matrice comprend au moins un mono-alcool, de préférence ayant moins de 9 atomes de carbone, tel que le tert-butanol, le diméthyl propanol, le cyclohexanol substitué ou non ; de la gélatine en solution aqueuse, de préférence de la gélatine de bœuf, de porc ou de poisson ; de l'agar-agar en solution aqueuse ou ses dérivés tels que l'agarose; au moins un acide carboxylique en C6 à C20 estérifié par un alcool ou par un alcool polyéthoxylé, tel que du PEG stéarate ; du glycérol éthérifié totalement ou partiellement par une chaîne polycarbonée en C6 à C20 et/ou par une chaîne polyéthoxylée; du glycérol estérifié totalement ou partiellement par un ou des acides carboxyliques à chaine en C6 à C20 et/ou polyéthoxylée, tel que du glycéryl monocaprylocaprate, du glycéryl dicaprylocaprate ou un mélange des deux; du glycérol éthérifié et estérifié totalement ou partiellement par une chaîne polycarbonée en C6 à C20 et/ou polyéthoxylée, tel que du caprylocaproyl polyoxyl-8 glycéride ; un oligomère ou un polymère du glycérol estérifié totalement ou partiellement par des acides carboxyliques à chaîne en C6 à C20 et/ou polyéthoxylés, tel que du polyglycéryl-3 dioléate ; un dérivé du propylène glycol estérifié par un acide carboxylique à chaîne polycarbonée en C6 à C20 et/ou polyéthoxylée, tel que du propylène glycol caprylate ; ou un mélange d'au moins deux d'entre eux, avec ou sans huile végétale, comme l'huile de soja ou l'huile d'amande. Des formulations contenant diverses quantités ou combinaisons des substances susmentionnées sont également envisagées. Par simplification, on pourra dire que la matrice est lipidique lorsqu'elle contient au moins un glycéride, au moins un ester d'acides gras en C6 à C20 et de glycérol, au moins un ester d'acides gras en C6 à C20 et de glycérol éthérifié, avec ou sans huile végétale, ou un mélange d'autre moins deux de ces composés. Par exemple, une telle matrice lipidique correspond au Gelucire^{®}48/16, Labrafac^{™} MC 60, Labrasol^{®} ALF, Geloil^{™} SC seuls ou en mélange, sans bien évidemment se limiter à ces composés ou ces mélanges de composés. Il est bien entendu qu'une telle matrice lipidique répondant aux critères physico-chimiques recherchés et évoqués ci-dessus pour résoudre le problème technique font partie de l'invention et que l'homme du métier saura choisir leur composition ou formulation.

De préférence, ces composés sont utilisés purs et non dilués. Il peut être avantageux de les utiliser en mélange afin d'ajuster et d'affiner la température ou point de fusion et la solubilité de la matrice. Par exemple dans une variante de réalisation avec le mélange Labrafac^{™}/Gelucire^{®}, ces composés sont utilisés à une teneur comprise entre 1% et 80% de Gélucire^{®} dans du Labrafac^{™} entre 20% et 99%, de préférence Labrafac MC60. Pour les polymères hydrosolubles que sont la gélatine, l'agar-agar et ses dérivés, ils sont dilués dans une solution aqueuse. En effet, la gélatine, l'agar-agar ou leurs dérivés se trouvent dans une quantité donnée dans une solution aqueuse, à savoir au maximum 10%, de préférence au maximum 5% et plus préférentiellement entre 2% et 5% du ou des composés dans la solution aqueuse. Lorsque ces composés sont choisis pour constituer la matrice du complexe, l'homme du métier saura trouver l'équilibre adapté pour que le complexe puisse se trouver sous deux états distincts : un état solide et un état liquide ou solubilisé. Il est impératif que le complexe puisse se trouver sous ces deux états et non pas uniquement sous une seule forme car ceci ne permettrait pas de répondre au problème technique de dispersion des particules d'extraction lors du transport du récipient les contenant et de disponibilités des particules d'extraction pour l'extraction des biomolécules susceptibles d'être contenues dans l'échantillon à tester. Si la quantité de ces composés est trop importante, la solubilisation sera affectée et si au contraire, la solution aqueuse est en quantité trop importante, la solidification du complexe sera altérée, voire impossible.

Dans une variante préférée de l'invention, la matrice est composée de tert-butanol ou d'un isomère du pentanol ou de mono et dicaprylocaprate de glycéryle en mélange avec un ester entre l'acide palmitique et/ou l'acide stéarique et un alcool polyéthoxylé, de préférence un PEG (n=32).

Dans une variante préférée de l'invention, l'isomère du pentanol est le 2,2 -diméthyl - 1-propanol ou le 2-méthyl-2-butanol et plus préférentiellement le 2,2 -diméthyl - 1- propanol.

Dans une variante de réalisation de la présente invention, il est possible de choisir une matrice disponible commercialement. Il peut s'agir par exemple de Gelucire^{®} (de préférence le Gelucire^{®} 48/16 ou 44/16), de Labrafac ^{™} (de préférence le Labrafac^{™} MC 60), de Labrasol^{®} (de préférence le Labrasol^{®} ALF), de Geloil^{™} (de préférence le Geloil^{™} SC) (Gattefossé, France) ou des matrices similaires qui sont disponibles commercialement par exemple le Kolliphor (BASF, Allemagne), le Capmul (Abitec, USA), le Miglyol (IOI Oleo), l'imwitor (Sasol) ou ces mêmes composés produits chez d'autres fabricants comme CRODA international, LUBRIZOL, SEPPIC...

Bien évidemment, il est possible d'avoir un complexe dont la matrice est constituée d'un mélange des composés listés ci-dessus. Il est donc compris que l'homme du métier saura adapter ces formulations à l'usage final attendu et au maintien des performances d'extraction des particules ainsi qu'en fonction du récipient ou support dans lequel le complexe est destiné à être fabriqué ou distribué.

Le ou les composés constituant la matrice sont adaptés aux particules d'extraction et permettent ainsi leur stockage à une température en-dessous du point de fusion de la matrice dans le complexe tout en conservant leur propriétés et performances de capture des biomolécules et en particulier des acides nucléiques. Il est ainsi avantageux que la structure des particules d'extraction et donc leurs propriétés de liaison et/ou de capture des biomolécules soient conservées car la durée de vie du test (récipient contenant le complexe selon l'invention et donnant un résultat reproductible et fiable) sera plus longue.

Comme évoqué plus haut, les particules d'extraction peuvent remplir leurs fonctions de capture des biomolécules susceptibles d'être contenues dans l'échantillon à tester lorsque la matrice du complexe de l'invention est sous forme liquide ou solubilisée. En effet, c'est sous cet état de la matrice que les particules d'extraction sont libres de leurs mouvements et qu'elles peuvent ainsi rencontrer les biomolécules de l'échantillon pour les capturer. Le complexe selon l'invention contenant des particules d'extraction des biomolécules d'un échantillon à tester et en particulier la matrice le constituant, quel que soit son état (solide, liquide ou solubilisée), est compatible avec la stabilité mais également avec les performances des particules d'extraction Selon l'invention, on considère que les particules sont performantes lorsqu'elles capturent au moins autant ou plus d'acides nucléiques que dans des conditions habituelles, c'est-à-dire sans complexe, qui représentent une référence (cf. tableau 1 de l'exemple 2 et tableau 2 de l'exemple 4 avec une comparaison à une référence liquide). De plus, le complexe et en particulier la matrice une fois solubilisée par l'ajout de la solution appropriée (eau, tampon aqueux, tampon de lyse de l'échantillon et/ou échantillon), elle se retrouve en contact avec le tampon de lyse et/ou l'échantillon à tester. Elle n'a pas d'effet inhibiteur ou autre interaction négative avec ces derniers. On peut ainsi dire que la matrice est compatible avec le tampon aqueux, le tampon de lyse et/ou l'échantillon à tester.

Au sens de l'invention, compatible signifie qui convient, qui n'interfère pas avec, qui cohabite avec sans entrer en conflit avec, qui ne perturbe pas, qui n'altère pas, qui n'entrave pas le bon fonctionnement, qui n'interagit pas avec, qui ne nuit pas à la structure de ou aux propriétés de, qui est inerte vis-à-vis de.

Par stabilité, on entend qui demeure dans un même état et dont la structure et les propriétés ne sont pas altérées, fragilisées ou modifiées ou de façon imperceptible ou minimales et acceptables. Ainsi lorsque l'on dit que la matrice est compatible avec la stabilité des particules d'extraction, on indique que la matrice ne nuit pas, n'altère pas ni la structure des particules d'extraction, ni leur capacité à capturer des biomolécules et en particulier des acides nucléiques. La matrice n'interagit donc pas de façon négative sur les propriétés physico-chimiques des particules d'extraction dont le rendement d'extraction n'est pas modifié.

De préférence, les particules d'extraction se lient aux biomolécules de l'échantillon lorsque la matrice est solubilisée par de l'eau, un tampon aqueux, un tampon de lyse et/ou l'échantillon à tester. On préférera ne pas trop chauffer l'échantillon pour ne pas dégrader ses biomolécules à capturer, ainsi bien que cela soit possible, il est préférable d'avoir une matrice solubilisée via une solution aqueuse et non une matrice liquide via la liquéfaction au point de fusion ou au-delà de celui-ci.

L'échantillon à tester contient ou est susceptible de contenir des analytes ou des biomolécules.

Par analyte, on entend tout micro-organisme ou fragment de micro-organisme (bactérie, virus, levure, champignon) mais également des acides nucléiques ou protéines de ces micro-organismes ou de l'hôte. L'hôte correspond à l'individu sur lequel on prélève l'échantillon. Il peut être humain ou non-humain.

Par biomolécule, on entend un composé ou une entité chimique pouvant être un acide nucléique (ADN ou ARN de tout type, ADN génomique, ADN complémentaire, ARN messager, ARN complémentaire, ARN de transfert, ARN mitochondrial, ADN de chloroplaste, ARN ribosomal, ADN plasmidique, ADN ou ARN viral, bactérien ou de levures, microARN, snoARN, siARN, ARNi, sous forme monocaténaire ou bicaténaire (cette liste n'étant pas exhaustive ou limitative) ou une protéine ou tout fragment de l'une de ces entités. De préférence, les particules d'extraction de la présente invention se lient à un ou des acides nucléiques contenus dans l'échantillon à tester. Les biomolécules peuvent être libres, circulantes, dans l'échantillon à tester, elles peuvent également être constituantes d'une structure biologique de l'échantillon ou contenues dans une de ces structures.

Dans une configuration préférée de l'invention, les biomolécules extraites par les particules d'extraction sont des acides nucléiques.

Par échantillon, on entend un échantillon ayant diverses origines comme les échantillons d'origine alimentaire, environnementale, humaine, vétérinaire ou cosmétique.

Parmi les échantillons d'origine alimentaire, on peut citer, de façon non-exhaustive, un échantillon de produits lactés (yaourts, fromages, etc.), de viande, de poisson, d'œuf, de fruit, de légume, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales. Tous ces échantillons, s'ils ne sont pas liquides, sont préalablement traités pour être sous forme liquide.

Tel qu'indiqué précédemment, l'échantillon peut être d'origine environnementale et peut consister, par exemple, en un prélèvement de surface, d'eau, etc.

L'échantillon peut également consister en un échantillon biologique, d'origine humaine ou animale, pouvant correspondre à des prélèvements de fluide biologique (urine, sang total ou dérivés tels que sérum ou plasma, crachat ou salive, pus, liquide céphalo-rachidien, moelle osseuse, etc.), de selles (par exemple diarrhées cholériques), de prélèvements de nez, de gorge, de peau, de plaies, de cheveux, d'organes, de tissus ou de cellules isolées, d'échantillons d'écouvillonnage, des prélèvements ou lavages broncho-alvéolaires, des biopsies. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, impliquant par exemple des étapes de mélange, de dilution ou encore de broyage, en particulier si l'entité de départ est à l'état solide.

L'échantillon analysé est, en général, susceptible de - ou suspecté de - contenir au moins une biomolécule représentative de la présence de microorganismes ou d'une maladie à détecter, caractériser ou suivre. L'échantillon peut être testé directement après son prélèvement mais il peut également être testé après congélation ou stockage au froid (par exemple à -20°C ou à 4°C). La congélation peut concerner l'échantillon lui-même ou l'échantillon déjà lysé (lysat)

De préférence, l'échantillon, quel qu'il soit, est sous forme liquide ou semi-liquide, c'est-à-dire qu'il peut contenir encore des matières ou substances solides, non-liquéfiées sans que cela ne gêne l'analyse qui doit en être faite.

L'échantillon à tester a un volume allant de quelques microlitres (environ 5µl) à plusieurs millilitres (10 ml), de préférence l'échantillon est compris entre 50µL et 1ml, plus préférentiellement entre 100µl et 200µl.

Il est possible, dans une variante de l'invention, que l'échantillon soit lysé au préalable, c'est-à-dire avant la mise en contact avec le complexe de l'invention et dans ce cas le procédé d'extraction des biomolécules selon l'invention ne comprend pas d'étape de lyse et il n'est pas utilisé de tampon de lyse.

Dans une variante préférée de l'invention, le procédé d'extraction des biomolécules de l'échantillon à tester comprend une étape de lyse de l'échantillon.

Le procédé d'extraction des biomolécules de l'échantillon à tester sera décrit plus loin.

Dans une variante de réalisation, le complexe de l'invention est destiné à être coulé ou versé à l'état liquide dans un récipient à une température comprise entre 20°C et 130°C en fonction de la matrice du complexe. De préférence, le complexe sera coulé ou versé à l'état liquide à une température comprise entre le point de fusion du ou des composés de la matrice et 90°C, de préférence entre ce point de fusion et 80°C. De cette façon, le complexe va épouser la forme du récipient qui le contient et ceci que ce soit sous sa forme liquide, solubilisée ou solide (une fois qu'il aura refroidi).

Ce complexe peut avoir plusieurs formes en fonction du récipient qui le reçoit ou le support qui le porte puisqu'il épouse la forme du récipient qui le contient. Lorsqu'il est mis en forme à l'état solide ou lors de son refroidissement (grâce à sa structure, malléable, visqueuse ou pâteuse) puis qu'il est distribué ultérieurement dans un récipient ou sur un support, il peut avoir différente forme, par exemple ellipsoïde, sphérique, cylindrique, cubique, parallélépipédique, conique, pyramidale, tétraédrique, octaédrique, de prisme triangulaire, rectangulaire, cuboïde, pentagonal, hexagonal ou tout autre forme. Il va adhérer au récipient ou support et présentera un volume plein, dépourvu de volume intérieur ou vide à la fois dans sa structure propre mais également dans son positionnement, agencement vis-à-vis du récipient qui le contient ou support qui le porte.

Dans un mode réalisation, l'invention concerne un support ou récipient comprenant un complexe conforme à l'invention ou obtenu comme décrit plus loin, dans lequel le complexe se présente sous la forme d'un volume plein à l'état solide, dépourvu de volume intérieur. Ce récipient peut avoir n'importe quelle forme du moment que le complexe selon l'invention puisse être coulé ou versé ou bien déposé à l'aide d'ustensile en son sein Il s'agit de préférence d'un récipient ou support permettant de faire un test biologique.

Le récipient de test peut être également appelé dispositif de test ou d'extraction ou contenant. Il peut s'agir d'un tube de type tube Eppendorf^{®}, d'un pot, d'une bouteille, d'une cartouche ou barrette (de type barrette Vidas^{®}), d'une poche souple de type FilmArray^{®}, avec un circuit microfluidique ou sans, d'une plaque multi-puits dont les puits sont tous de tailles identiques ou de tailles différentes ou tout type de récipient adapté pour contenir un complexe selon l'invention et dans lequel un test de biologie moléculaire (extraction, amplification et détection) et tout particulièrement une extraction peut être réalisé(e).

Le volume du récipient est adapté aux volumes choisis pour réaliser le test et en particulier le volume de l'échantillon à tester et le volume du complexe selon l'invention.

Le récipient est de préférence en plastique mais il peut aussi être en métal, en verre ou en tout matériau inerte ou n'interagissant pas avec l'échantillon et les substances utilisés pour le test (tampon, enzyme, particules d'extraction, matrice du complexe...)

Le récipient peut être fermé ou ouvert. Dans un mode de réalisation préféré de l'invention, le récipient est fermé. La fermeture peut se faire par un bouchon, un vissage, un opercule ou foil qui peut être une feuille d'aluminium, un film plastique ou un multicouches. Généralement, l'opercule est de faible épaisseur permettant de fermer de manière étanche, par thermoscellage ou scellage adhésif.

Le récipient de l'invention contient le complexe lorsqu'il s'agit d'un récipient n'ayant qu'un seul puits mais lorsqu'il comprend plusieurs puits, il peut contenir des réactifs qui vont permettre les différentes étapes du procédé d'extraction selon l'invention ou les différentes étapes du procédé de détection selon l'invention. Ainsi, le récipient peut comprendre des solutions de lavages, un tampon d'amplification comprenant entre autres des nucléotides et une enzyme polymérase, un réactif de détection et/ou un tampon de lyse. Ainsi, la présente invention couvre également un nécessaire de diagnostic comprenant au moins un complexe conforme à l'invention et un récipient ou un support dans ou sur lequel le diagnostic sera réalisé.

Pour pallier les problèmes évoqués plus haut concernant la dispersion des particules d'extraction de biomolécules dans un récipient et en particulier lors du transport de ce dernier, les inventeurs ont eu l'idée originale de combiner l'utilisation d'une matrice choisie, aux propriétés particulières pour immobiliser des particules d'extraction à une localisation donnée du récipient de test biologique. La présente divulgation couvre donc un tel procédé d'immobilisation de ces particules à une localisation donnée dans un récipient ou sur un support, consistant à mettre en œuvre le complexe, tel que défini ci-dessus ou obtenu selon le procédé de fabrication ou le procédé de distribution de l'invention décrit plus bas.

### PROCEDE DE FABRICATION ET DE DISTRIBUTION DU COMPLEXE

L'invention a également pour objet un procédé de fabrication d'un complexe tel que défini ci-dessus comprenant les étapes suivantes :
a) Prendre des particules d'extraction de biomolécules d'un échantillon à tester,
b) Lorsqu'il est présent, retirer le liquide dans lequel les particules d'extraction de biomolécules d'un échantillon à tester sont contenues, ledit liquide pouvant être un tampon de stockage, un solvant, un tampon aqueux, de l'eau ou une matrice au moins partiellement solubilisée,
c) Mettre en contact lesdites particules d'extraction de biomolécules avec une matrice telle que définie pour l'invention, à une température à laquelle ladite matrice est liquide pour former un mélange ou une suspension de particules d'extraction des biomolécules à une concentration donnée dans la matrice liquide,
c') Optionnellement, réitérer l'étape b) et c),
d) Refroidir ou laisser refroidir naturellement le mélange ou suspension en vue d'obtenir le complexe sous forme solide.

Généralement, les particules d'extraction de biomolécules sont stockées en suspension dans un tampon de stockage. Elles peuvent également se trouver dans un autre tampon, un autre milieu aqueux ou dans un solvant adapté. Pour préparer le complexe selon l'invention, il convient de prélever une quantité déterminée de particules d'extraction. Cette quantité va dépendre de la taille ou volume défini du complexe à réaliser. En général, on prélève une quantité allant de 20µl à 200µl de cette suspension de particules d'extraction.

Si les particules n'ont pas besoin d'être magnétiques et magnétisées, le surnageant de la suspension ou liquide dans lequel les particules d'extraction sont contenues (par exemple, le tampon de stockage) sera retiré par pipetage. Il pourra également être retiré après décantation, après centrifugation adaptée ou filtration. Les outils et réglages sont bien connus de l'homme du métier et seront adaptés en fonction du type de particule d'extraction choisi, du volume et du besoin. Bien évidemment, le retrait de ce liquide ou surnageant n'entraine pas pour autant le dessèchement ou le séchage des particules d'extraction qui doivent conserver leurs propriétés et performances dans le complexe.

Si cette suspension de particules d'extraction n'est pas magnétisée et a besoin de l'être, on peut la magnétiser avec des appareils adaptés, par exemple sur un portoir aimanté doté d'aimants Neodyme/bore, un électroaimant. Cette magnétisation se fait pendant une durée adaptée allant de quelques secondes (environ 10 secondes) à quelques minutes (environ 5 minutes). Les particules d'extraction une fois magnétisées seront immobilisées grâce à un champ magnétique et le surnageant ou liquide dans lequel elles sont contenues pourra être retiré selon les mêmes techniques qu'utilisées pour des particules non magnétisées. De préférence, le surnageant sera retiré par pipetage ou pompage.

Le surnageant ou liquide est remplacé par une matrice donnée, à une température à laquelle elle est sous forme liquide pour former un mélange ou une suspension de particules d'extraction des biomolécules à une concentration donnée dans la matrice liquide. Cette température a été exposée plus haut et correspond à la température du point de fusion du ou des composés de cette matrice ou au-delà de cette température et ce jusqu'à 200°C maximum, de préférence jusqu'à 130°C, plus préférentiellement jusqu'à 100°C et encore plus préférentiellement jusqu'à 80°C. Le liquide ou surnageant est donc remplacé par un volume adapté de matrice, de préférence par un volume équivalent au volume de surnageant retiré. Bien évidemment, en fonction du besoin et de la concentration finale de particules d'extraction souhaitée, le volume de matrice ajouté sera adapté. Dans un mode préféré de réalisation de l'invention, la suspension finale de particules d'extraction est à 20 mg/ml dans la matrice désirée. Il est bien entendu que le surnageant peut être remplacé par un mélange de matrices adaptées au complexe selon l'invention et telles que définies précédemment.

Dans une variante de réalisation de l'invention, il ne sera pas obligatoire que la matrice soit totalement à l'état liquide pour former une suspension avec les particules d'extraction mais il suffira que le chauffage de la matrice ou que la température de la matrice soit tel(le) que la matrice ait une consistance ou une fluidité suffisante pour former une suspension avec les particules d'extraction. L'homme du métier saura définir de tels critères.

Dans un mode de réalisation préféré de l'invention, le remplacement du surnageant (par exemple, tampon de stockage) est effectué à plusieurs reprises, de préférence à deux reprises afin d'être certains de ne plus avoir de milieu aqueux résiduel (tel que le tampon de stockage) avec les particules d'extraction et uniquement et presque uniquement de la matrice telle que définie précédemment. C'est pour que la matrice puisse être sous forme solide ou non solubilisée que les étapes b) et c) du procédé de fabrication sont préférentiellement réitérées. Ceci permet d'être certain que, dans le mélange formé par les particules d'extraction et la matrice, il ne reste pas de milieu aqueux (tampon de stockage, eau, tampon de lyse ou tampon aqueux) ou alors une quantité minimale n'entrainant pas la solubilisation (même partielle) de la matrice. Ainsi, ceci permet de s'assurer que la matrice puisse être solidifiable lorsque la température sera redescendue en-dessous du point de fusion du ou des composés de la matrice. Dans cette variante du procédé de fabrication du complexe, l'étape c') consiste à retirer le liquide environnant les particules d'extraction et à ajouter une quantité déterminée et adaptée de matrice dans son état liquide, de préférence, ou solide. Si la matrice est ajoutée aux particules d'extraction à l'état solide, il conviendra de chauffer le tout à une température d'au moins le point de fusion de la matrice pour que celle-ci se liquéfie et forme une suspension avec les particules d'extraction. La mise en contact de la matrice et des particules d'extraction dépourvues de tout milieu aqueux se fait de préférence lorsque la matrice est directement sous forme liquide, c'est à-dire qu'elle a été chauffée préalablement à une température égale ou supérieure au point de fusion du ou des composés la constituant. Dans une autre variante de réalisation, il est possible de mettre en contact les particules d'extraction avec la matrice sous forme solubilisée puis d'effectuer l'étape de retrait du liquide environnant les particules d'extraction et ainsi retirer toute trace de milieu aqueux. Ensuite, une quantité définie et adaptée sera ajoutée aux particules d'extraction. Quoiqu'il en soit, au final c'est toujours la forme liquide de la matrice (liquide directement, solide puis chauffée ou bien liquide solubilisée) qui est mise en contact avec les particules d'extraction.

La mise en contact des particules d'extraction et de la matrice choisie, à l'état liquide, donne un mélange ou une suspension qui est, de préférence, agitée de sorte à obtenir une solution homogène liquide ou fluide. Bien évidemment, des colorants, conservateurs ou tout autre composant nécessaire et adapté au complexe selon l'invention peuvent être ajoutés dans cette solution homogène sans que cela nuise aux propriétés du complexe et des particules incluses dans celui-ci. De préférence, il n'est rien ajouté, la solution homogène n'est constituée que de particules d'extraction et de matrice.

La suspension est ensuite laissée à refroidir naturellement à l'air libre ou est refroidie par tout moyen adapté (ventilateur, réfrigérateur, congélateur, chambre froide ou autre moyen) de sorte que sa température passe en -dessous du point de fusion de la matrice et que celle-ci et, par conséquence, le complexe de l'invention se trouve alors dans son état solide. Il est à noter que le procédé de fabrication du complexe selon l'invention ne comprend pas d'étape de séchage ou de déshydratation car ces étapes ont pour conséquence d'altérer les performances de particules contenues dans le complexe.

Il sera bien compris de ce qui précède que le complexe de l'invention peut se trouver sous trois formes distinctes : une forme solide quand la matrice est à une température en-dessous du point de fusion du ou des composés la constituant, une forme liquide quand la matrice est au-dessus de la température de ce point de fusion et enfin une forme solubilisée quand elle est en contact avec un milieu aqueux quelle que soit la température. Tant que la matrice n'a pas été en contact avec un milieu aqueux, elle peut être solide ou liquide en fonction de la température. Une fois qu'elle est en contact avec un milieu aqueux, elle est nécessairement sous forme liquide solubilisée qu'on appellera uniquement forme solubilisée pour qu'il n'y ait pas de confusion. Sous cette forme solubilisée, elle ne pourra pas redevenir solide, même si la température est en-dessous du point de fusion du ou des composés de la matrice. Pour redevenir solide, il faudrait retirer tout milieu aqueux.

Après refroidissement, le complexe selon l'invention dans son état solide est ainsi fabriqué. Il pourra être fabriqué *in situ,* c'est-à-dire qu'il ne sera pas ensuite déplacé ou distribué dans un autre contenant ou support. Il pourra donc être laissé sur place. De préférence le complexe selon l'invention est distribué dans un récipient ou sur un support selon le procédé de distribution du complexe comprenant au moins une des étapes suivantes :
A) Lorsque le complexe est à l'état liquide, le couler, le verser ou le dispenser à l'aide d'un ustensile adapté sur un support ou dans un récipient, de préférence un récipient d'analyse biologique,
B) Refroidir ou laisser refroidir naturellement le complexe distribué de sorte à ce qu'une fois à l'état solide le complexe ait un volume plein, dépourvu de volume intérieur et qu'il se présente sous la forme d'un culot,
   ou
C) Lorsque le complexe est sous forme solide, déposer au moins une fraction dudit complexe sur le support ou dans le fond du récipient de sorte que qu'il forme un complexe ayant un volume plein, dépourvu de volume intérieur.

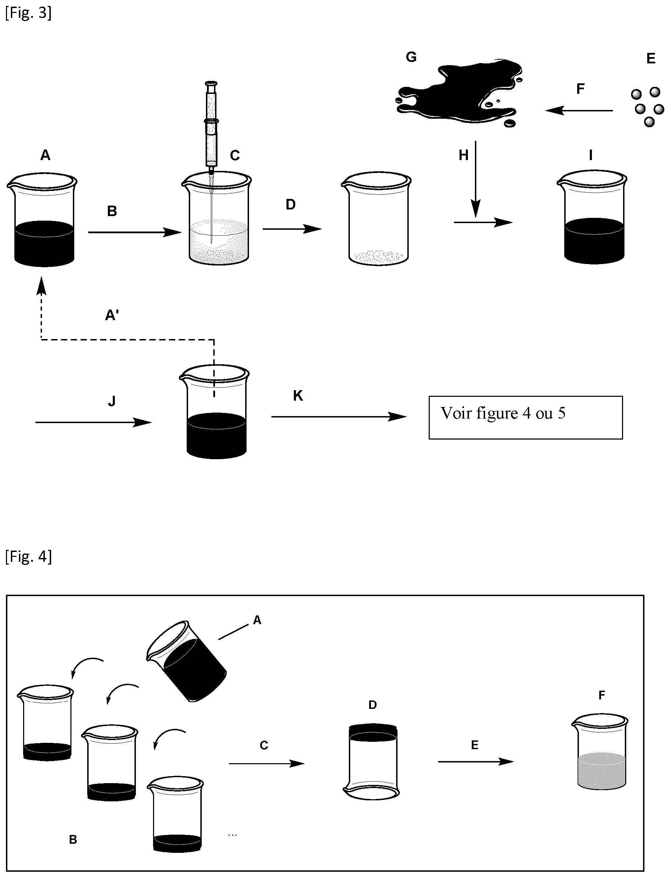

Le complexe une fois chauffé à la température de fusion du ou des composés de la matrice se présente sous forme liquide et peut être distribué sur un support choisi ou dans un récipient. Cette distribution peut se faire en versant le mélange liquide dans un ou plusieurs autres récipients (cf. figure 4) adaptés tels que décrit précédemment. Ce versement peut se faire par tous les moyens adaptés connus de l'homme du métier qui choisira le moyen le plus adéquat en fonction du récipient primaire où a été fabriqué le mélange et le(s) récipient(s) secondaire(s) où le complexe sera distribué et où il pourra se solidifier. Il peut s'agir par exemple d'un récipient avec un bec verseur (cf. figure 4), de l'utilisation d'un entonnoir, d'une pipette, d'une seringue... Ces modes de réalisation supposent que le complexe soit distribué lorsqu'il est encore dans un état liquide ou semi-liquide c'est-à-dire suffisamment fluide pour qu'il puisse être coulé, versé ou dispensé à l'aide d'un ustensile ou d'un instrument adapté pour cette distribution et le dosage précis et ceci de façon manuelle, semi-automatisée ou totalement automatisée. Par exemple, on pourra utiliser une spatule, une cuillère, une seringue, un robot pipeteur...

Dans une varianté préférée de réalisation, le complexe est distribué à l'état liquide à l'aide d'un pipeteur automatique pour pouvoir avoir une distribution précise, propre et reproductible.

Dans une variante de réalisation, il est possible d'avoir un complexe solide que l'on chauffe au-delà du point de fusion de la matrice pour rendre le complexe à l'état liquide puis que l'on distribue dans un récipient ou un support avant que l'étape de refroidissement du procédé de fabrication du complexe n'ait lieu, il y a donc dans cette variante une combinaison du procédé de fabrication et de distribution du complexe.

Lorsque le complexe est distribué à l'état liquide, il est ensuite refroidi de façon naturelle ou artificielle comme ceci est décrit précédemment pour la fabrication du complexe de sorte à obtenir un récipient contenant un complexe à l'état solide qui présente un volume plein, sans volume intérieur ou vide intérieur. De préférence, ce complexe est sous forme de culot dans le récipient. Il épouse non seulement les contours ou parois du récipient ou support et y adhère.

Dans une variante de réalisation du procédé de distribution, le complexe à l'état solide est fractionné et une fraction est déposée sur le support ou dans le récipient de sorte que cette fraction de solide présente un volume plein sur le support ou dans le récipient et y adhère. Cette fraction peut être réalisée à l'aide de tout ustensile, dispositif ou instrument adapté, telle que par exemple une lame, un cutter rotatif, une spatule, une poinçonneuse, une perforatrice...tout moyen connu de l'homme du métier qui choisira le plus adéquat en fonction du complexe et des formes du récipient ou support primaire et secondaire (où la distribution sera faite). De la même façon, la distribution de cette fraction se fera à l'aide de tout ustensile, dispositif ou instrument adapté que l'homme du métier sera à même de choisir en fonction des contraintes et des besoins.

Au final, dans le récipient ou sur le support, le complexe selon l'invention se trouve à l'état solide et présente un volume plein sans vide intérieur tel que ceci a été défini précédemment. Il y adhère dans le fond ou sur la paroi. Dans une variante préférée de réalisation, le complexe selon l'invention est distribué dans un récipient et de préférence dans un récipient dans lequel un test ou une analyse biologique sera effectuée.

Il est bien évident que le récipient ou support est adapté à la taille du complexe. Ce procédé de distribution consiste à doter un récipient ou un support d'un complexe selon l'invention.

Lorsque le récipient contient plusieurs puits, un seul des puits contient un complexe. Le complexe peut être ajouté au récipient ou support longtemps à l'avance. Ceci est le cas lorsque le récipient est dit prêt-à-l'emploi et que le manipulateur ou l'automate réalisant le test n'a plus qu'à ajouter l'échantillon à tester. Comme il a été vu précédemment, le complexe inclus dans le récipient empêchera les particules d'extraction contenues dans le complexe de se disperser sur les parois du récipient lors du transport ou la manipulation de celui-ci. La distribution du complexe dans le récipient se fait de façon automatisée ou manuellement. Le mode automatisé sera le mode préféré car il fait gagner du temps, il est plus reproductible et n'entraine pas de troubles musculosquelettiques pour le manipulateur. Lorsque le remplissage du récipient est effectué, ce dernier est operculé ou refermé afin de faciliter son transport.

Dans une variante de réalisation, la distribution du complexe dans le récipient de test ou le récipient d'extraction est faite par le manipulateur ou l'automate de test/ l'instrument de test directement au moment où le test est fait, c'est-à-dire juste quand l'échantillon à tester est à disposition. Ce mode peut être utilisé lorsque le récipient n'a pas de couvercle, ou d'opercule et que le transport du récipient contenant déjà le complexe aurait été compliqué. Ce mode de distribution garde l'avantage de ne pas permettre la dispersion des particules d'extraction sur les parois du récipient lorsque l'échantillon ou un tampon aqueux ou de lyse est ajouté (splashing) mais également de ne pas contaminer l'automate ou la paillasse du manipulateur.

Que ce soit pour le procédé de fabrication et/ou de distribution du complexe selon l'invention, l'homme du métier saura choisir les techniques et composants adaptés en fonction du complexe à réaliser et du récipient ou support destiné à le recevoir.

### PROCEDE D'EXTRACTION ET PROCEDE DE DETECTION DE BIOMOLECULES

La présente invention couvre un procédé d'extraction de biomolécules contenues dans un échantillon à tester comprenant :
- Une étape de solubilisation du complexe tel que défini précédemment, tel qu'obtenu selon le procédé de fabrication défini précédemment ou tel que contenu sur un support ou dans un récipient tel qu'exposé précédemment, par la mise en contact dudit complexe avec un tampon aqueux, un tampon de lyse et/ou l'échantillon à tester lysé ou non lysé,
- Optionnellement si cette étape n'a pas encore eu lieu, une étape de mise en contact du complexe solubilisé avec l'échantillon à tester lysé ou non lysé,
- Optionnellement si l'échantillon à tester n'a pas déjà été lysé, une étape de lyse de l'échantillon à tester,
- Une étape de capture des biomolécules de l'échantillon à tester par les particules d'extraction libres de leurs mouvements dans de la matrice solubilisée, les biomolécules étant de préférence des acides nucléiques,
- Optionnellement, une étape de lavage des biomolécules capturées par les particules d'extraction,
- Optionnellement, une étape d'élution des biomolécules capturées par les particules d'extraction.

Par extraction, on entend une technique permettant d'isoler des biomolécules à partir d'un échantillon quel qu'il soit, par exemple, l'isolement d'ADN ou d'ARN à partir de cellules eucaryotes, procaryotes, animales humaines, de micro-organismes ou d'un tissu. Ainsi, l'extraction au sens de l'invention inclut la lyse de l'échantillon et du complexe de l'invention et la purification des biomolécules. La purification comprend elle-même l'adsorption ou capture, le lavage et l'élution des biomolécules et/ou des micro-organismes. La capture consiste à adsorber les biomolécules et/ou micro-organismes sur les particules d'extraction et l'élution à la désorption ou libération de ces derniers des particules d'extraction.

L'échantillon à tester peut être prélevé quelques heures ou jours avant le test à réaliser ou extemporanément. Pour être testé, il doit être ajouté au récipient dans lequel le test/diagnostic va se faire. Comme vu précédemment, le récipient peut être une cartouche, une plaque, un tube, une barrette...Lorsque le récipient contient plusieurs puits ou qu'il y a plusieurs récipients, l'échantillon est ajouté dans un puits où se trouve déjà le complexe selon l'invention. Le procédé d'extraction de l'invention peut comprendre une étape d'ajout du complexe selon l'invention dans un récipient mais également une étape d'ajout de l'échantillon à tester ou d'un échantillon à tester déjà lysé dans le récipient de test.

Dans une variante de réalisation, il est prévu que l'échantillon soit ajouté au récipient en premier et que le complexe soit ajouté à l'échantillon déjà présent dans le récipient. Dans ces deux cas, c'est l'échantillon qui va solubiliser le complexe mais il peut être prévu également qu'on ajoute ensuite de l'eau, un tampon aqueux et/ou un tampon de lyse à ce mélange et que cela participe également à la solubilisation du complexe.

Il est également possible que le complexe soit présent ou ajouté dans un puits du récipient ou dans un récipient donné et que l'échantillon soit ajouté dans un autre puits du récipient ou dans un autre récipient ne contenant pas de complexe. Dans ce cas, ce n'est qu'une fois le complexe solubilisé par de l'eau ou un tampon adapté (tampon de lyse ou un autre tampon aqueux) que les particules d'extraction sont libérées dans le puits où se trouvait le complexe non solubilisé et qu'elles sont ensuite transportées vers le puits ou le récipient qui contient l'échantillon. Dans ce cas, si l'échantillon n'avait pas été lysé avant l'ajout au récipient ou avant la mise en contact avec les particules d'extraction, il est nécessaire d'ajouter une étape de lyse de l'échantillon.

Il est également possible que ce soit l'échantillon déjà lysé ou ayant été lysé dans un puits du récipient ou dans un récipient donné qui soit déplacé vers le puits contenant le complexe solubilisé. Il sera donc compris que l'échantillon et le complexe peuvent se trouver dans le même puits d'un récipient ou dans des puits ou récipients distincts et se retrouver ensuite ensemble. S'il n'y a pas eu d'étape de lyse de l'échantillon, il conviendra d'en faire une avant l'étape d'extraction.

Dans une variante de réalisation de l'invention, l'échantillon peut avoir été lysé au préalable et avoir été congelé ou non. Dans ce cas, il n'y a pas d'étape de lyse supplémentaire et la solubilisation du complexe se fait soit par l'échantillon lui-même, soit par de l'eau, un tampon aqueux ou un tampon de lyse dans un puits ou un récipient distinct de celui accueillant l'échantillon.

La solubilisation du complexe se fait de façon chimique par de l'eau, un tampon aqueux ou l'échantillon lui-même. Généralement, la nature de la matrice ne nécessite pas d'action mécanique mais cette dernière peut juste être utilisée pour mélanger de l'agent solubilisant (l'eau, un tampon aqueux ou l'échantillon et accélérer le processus de solubilisation du complexe

Dans une varianté préférée de l'invention, le complexe est solubilisé dans un récipient ou un puits qui est distinct du récipient ou du puits contenant l'échantillon et les particules d'extraction rendues libres par la solubilisation du complexe sont transportées jusqu'au récipient ou au puits contenant l'échantillon lysé.

Dans une variante de réalisation du procédé d'extraction, le complexe est chauffé de sorte qu'il devienne liquide puis il est mis en contact
- avec un tampon aqueux tel que défini précédemment (eau, tampon de lyse ou autre tampon aqueux) puis enfin avec l'échantillon non lysé, ou
- avec un mélange du tampon de lyse et de l'échantillon lysé, ou
- avec l'échantillon déjà lysé. Dans ce dernier cas, on peut rajouter du tampon de lyse au mélange échantillon lysé et complexe pour avoir une capture optimale des acides nucléiques.

La lyse de l'échantillon peut être mécanique, chimique ou une combinaison des deux. Diverses techniques peuvent être utilisées à des fins de lyse, par exemple, le broyage avec éléments solides, l'homogénéisation à haute pression, la sonication, l'ultrasonication, le choc osmotique, la lyse par congélation/décongélation, l'utilisation de composés chimiques comme des détergents, des enzyme lytiques (lyzozyme, proteinase K...)....Plus particulièrement, la lyse par technique de broyage est une technique qui consiste à détruire la paroi cellulaire grâce à des contraintes de cisaillement créées par des éléments solides qui sont préférentiellement des particules ou billes. Les particules peuvent être en verre, silice, céramique, zirconium ou en encore métal, magnétiques ou non. Ces dernières sont agitées dans un contenant hermétique en présence des cellules à broyer. L'agitation peut être interne ou externe au contenant et générée par des pales, par magnétisation, par un vortex, par exemple. Ces différents moyens sont connus de l'homme du métier et sont communément utilisés en biologie moléculaire et notamment pour lyser les membranes des cellules, fragiliser des structures biologiques.

La lyse de l'échantillon peut être chimique et se fait grâce au tampon de lyse dont les composants vont permettre de défaire les structures chimiques des membranes des cellules pour libérer leur contenu (ADN, ARN, protéines, organites) et de solubiliser les protéines, sans les dégrader. La lyse chimique se fait via des détergents ioniques, tel que le Triton, qui préservent les activités de protéines, ou non-ioniques, tel que le SDS, qui perturbent et rompent les membranes et les protéines. La lyse chimique peut également se faire avec des enzymes tel que le lyzozyme, la protéinase K.

Une combinaison d'une lyse mécanique et chimique peut être envisagée et aura donc pour effet d'être plus complète et rapide. Dans la présente invention, on préfèrera une combinaison des deux types de lyse.

La lyse de l'échantillon en présence du complexe solubilisé n'altère pas les propriétés des particules d'extraction.

Une fois que le complexe est solubilisé, les particules d'extraction ne sont plus immobilisées par la matrice à l'état solide et deviennent donc libres de leurs mouvements. Ainsi, une fois que le complexe solubilisé est mis en contact avec l'échantillon lysé, si cette étape n'avait pas déjà eu lieu, l'étape de capture des biomolécules dudit échantillon par ces particules d'extraction peut donc se faire.

Après l'étape de capture des biomolécules, on peut procéder à une ou plusieurs étapes de lavage en présence d'un tampon de lavage, permettant l'élimination des éléments contaminants sans détacher les biomolécules (acides nucléiques et/ou protéines) des particules d'extraction. L'étape de lavage peut, par exemple, comprendre une étape de lavage dans de l'eau ou un tampon de lavage approprié, par exemple une solution faiblement chargée en sel contenant éventuellement un alcool et/ou une étape de lavage en présence d'un alcool afin d'éliminer les sels. Dans un mode préféré de réalisation de l'invention, au moins une étape de lavage est effectuée. Le tampon de lavage peut par exemple comprendre du citrate, de l'acétate de sodium ou du MES, éventuellement dans des concentrations comprises entre 10 et 100 mM. Il peut être une solution de lavage à base d'éthanol, par exemple Éthanol : 70-80%, EDTA : 1 mM, ou une solution de lavage à base de sels, par exemple NaCl : 100-200 mM et EDTA : 1 mM, ou une solution de lavage à base de guanidine, par exemple hydrochlorure de Guanidine : 50-100 mM, Tris-HCl : 10 mM (pH 7.5-8.0) et EDTA : 1 mM ou tout autre solution de lavage adaptée et connue de l'homme du métier.

Le cas échéant, on peut procéder ensuite à une étape d'élution des biomolécules (acides nucléiques et/ou protéines). L'étape d'élution consiste à libérer les biomolécules retenues sur les particules d'extraction. On utilise en général un tampon à pH basique, par exemple un pH compris entre 8 et 10, et de faible force ionique, tel que du tampon TrisHCl ou borate. Elle peut se faire de préférence à une température comprise entre 50°C et 90°C. Lorsque les biomolécules sont des acides nucléiques, l'élution se fait de façon préférentielle avec des produits commerciaux tels que les produits de la gamme d'extraction NucliSENS^{®} easyMAG^{®} (bioMérieux, France). Ces conditions sont des conditions classiques permettant de conserver l'intégrité des acides nucléiques et sont des conditions standards.

Dans un mode préféré de l'invention, le procédé d'extraction des biomolécules comprend une étape d'élution et encore plus préférentiellement il comprend au moins une étape de lavage et une étape d'élution.

Dans un mode de réalisation particulièrement préféré de l'invention, dans le procédé d'extraction, les biomolécules extraites sont des acides nucléiques.

Les acides nucléiques ainsi purifiés par le procédé décrit ci-dessus (avec ou sans étape de lavage et/ou d'élution) sont utilisables en particulier pour des applications d'amplification et de détection in vitro.

La présente divulgation couvre également un procédé de détection des biomolécules contenues ou susceptibles d'être contenues dans l'échantillon à tester. Ce procédé de détection correspond au procédé d'extraction des biomolécules d'un échantillon tel que décrit ci-dessus auquel sont ajoutées les étapes suivantes :
- Optionnellement, une étape d'amplification des acides nucléiques lorsque les biomolécules extraites à l'issue du procédé d'extraction ci-dessus sont des acides nucléiques, de préférence l'amplification est réalisée par PCR,
- Une étape de détection des biomolécules par des méthodes appropriées.

Alternativement, on procède directement à l'étape de détection ou d'amplification (lorsque les biomolécules sont des acides nucléiques) après les étapes de lyse, capture et éventuellement de lavage et élution. De préférence, le procédé comprend une étape de lavage et d'élution ainsi qu'une étape d'amplification et de détection. Cette d'amplification et détection peut être réalisée directement sur les particules d'extraction ayant capturé les biomolécules sans étape d'élution préalable. Ceci se fait plus préférentiellement encore sur une particule d'extraction magnétique ou non.

Par amplification ou réaction d'amplification, on entend toute technique d'amplification d'acides nucléiques bien connue par l'homme du métier

Généralement, l'étape d'amplification est réalisée car la quantité des acides nucléiques à détecter et/ou quantifier est très faible et qu'il est nécessaire de passer par une phase d'amplification de ces derniers pour les détecter et/ou les quantifier afin de donner un diagnostic précis. Sans phase d'amplification et si la quantité initiale d'acides nucléiques contenus dans l'échantillon est faible, il est tout à fait possible d'obtenir des résultats erronés concluant à l'absence d'un acide nucléique cible dans un échantillon alors qu'en réalité il est présent dans ce dernier mais en quantité tellement faible que les techniques utilisées ne permettent pas de le détecter.

Dans un mode de réalisation spécifique, l'amplification du procédé de détection selon l'invention est mise en œuvre à l'aide d'une ADN polymérase, telle que la Taq polymérase, la Pfu polymérase, la T7 polymérase, le fragment Klenow d'ADN polymérase d'E. coli et/ou une transcriptase inverse, ou tout autre polymérase adaptée.

Dans un autre mode de réalisation, l'amplification du procédé de détection est une réaction d'amplification en chaine par polymérase (réaction PCR), bien connue de l'homme du métier. Le protocole PCR comprend par exemple, 20 à 40 cycles, chaque cycle comprenant au moins (i) une phase de dénaturation de l'ADN à amplifier à une température généralement comprise entre 90°C et 95°C, (ii) une phase d'hybridation des amorces avec l'ADN à amplifier à une température généralement comprise entre 55°C et 65°C et (iii) une phase d'extension à une température généralement comprise entre 68°C et 75°C.

Des variantes de procédés d'amplification nucléique par PCR peuvent également être mises en œuvre. On citera en particulier la PCR emboitée (Nested PCR), les PCR quantitatives (ou qPCR), semi-quantitatives ou temps réel, les PCR « error-prone », ou la PCR après transcription inverse (RT-PCR), ou la digital PCR. Il est également possible de mettre en œuvre d'autres techniques d'amplification telles que LAMP, NASBA, TMA, RPA, LCR, RCR, 3SR, RCA, SDA ou toutes techniques d'amplification des acides nucléiques connues de l'homme du métier.

Les méthodes de détection classiques utilisables sont toutes celles largement connues de l'homme du métier. Par exemple, on peut citer de façon non exhaustive des marquages radioactifs; des marquages froids: colorimétrie, fluorescence, chimioluminescence ; des hybridations moléculaires ; des Southern Blot ; des Northern Blot ; des Dot Blot ou de l'hybridation in situ. De façon préférentielle, on utilise une détection avec une sonde détection ou un composé fluorigène de type « intercalant » dont la fluorescence est exaltée en présence d'ADN.

Par sonde de détection ou sonde, on entend une séquence nucléique d'un enchaînement nucléotidique de quatre bases de types différents choisis parmi le groupe d'adénine, thymine, guanine, uracile, cytosine, qui est capable de s'hybrider spécifiquement sur un amplicon et comportant au moins un marqueur. La sonde peut être une sonde de forme arrondie (cf. WO10001074), une balise moléculaire (autrement appelée Molecular Beacon), une sonde TaqMan^{®} ou une sonde FRET (cf. EP23307400.4). Ces trois derniers types de sondes sont bien connus de l'homme du métier. Ces sondes peuvent éventuellement être constituées en tout ou partie de nucléotides modifiés. Chaque sonde comporte un marqueur et éventuellement un quencher. Parmi ces sondes, préférentiellement, on utilise des sondes émettant une fluorescence lorsqu'elles s'hybrident à la séquence complémentaire, des sondes décrites par Tyagi & Kramer (Nature Biotech, 1996, 14 :303-308), communément connues sous le nom de «molecular beacons» ou des kits commerciaux comme, par exemple, des kits de la gamme R-gene^{®} de chez Argène (bioMérieux, Verniolle, France). Il est possible de faire la détection en temps réel ou à la fin de la réaction.

Lorsque les biomolécules à détecter sont des protéines, des techniques largement connues de l'homme du métier peuvent être utilisées pour la détection et la quantification des protéines. Parmi celles-ci, on peut citer par exemple le Western Blot qui utilise aussi un anticorps et la colorimétrie, la chimioluminescence ou la fluorescence; l'ELISA (Enzyme-Linked Immunosorbent Assay); la spectrophotométrie UV; des méthodes colorimétriques (BCA (Bicinchoninic Acid) ou Bradford); la cytométrie en flux et la fluorimétrie avec par exemple la GFP (Green Fluorescent Protein).

Les tests réalisés de détection avec ou sans amplification d'acides nucléiques cibles ainsi que les tests de détection des protéines avec les complexes selon l'invention donnent d'excellents résultats.

Il est bien évident que l'homme du métier saura choisir les techniques les plus adaptées au besoin pour l'extraction et la détection des biomolécules susceptibles d'être contenues dans un échantillon.

L'invention sera mieux comprise à l'aide des figures annexées et des exemples ci-dessous qui se rapportent à des modes de réalisation selon la présente invention, donnés à titre d'exemples non limitatifs.

### DESCRIPTION DES FIGURES

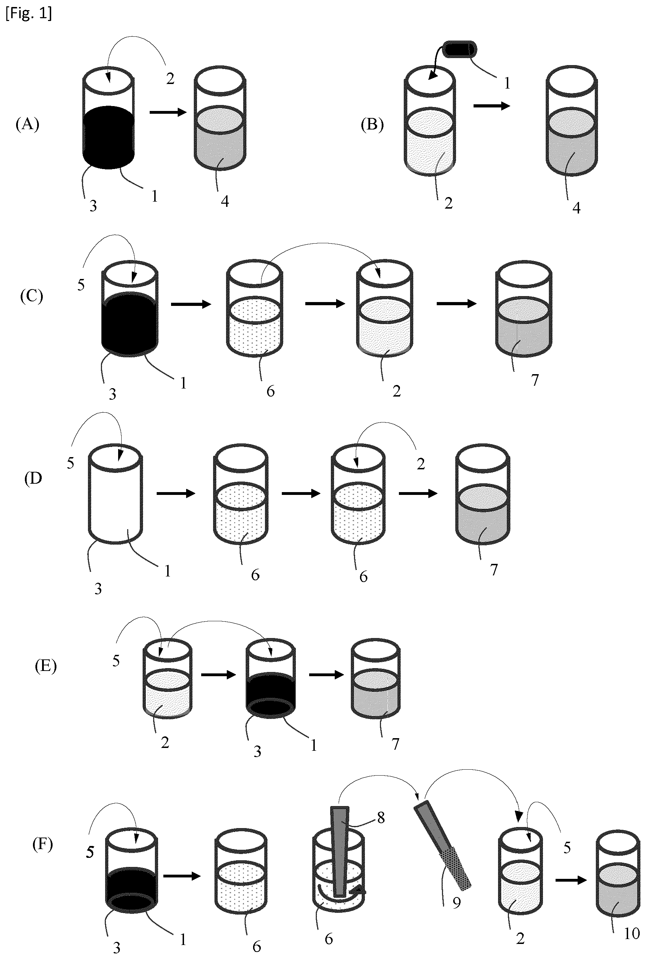

[Fig. 1] est un schéma de variantes du procédé de solubilisation du complexe de l'invention dans un ou plusieurs récipients pouvant être utilisés pour le procédé d'extraction des biomolécules issues d'un échantillon à tester. En (A), on voit un récipient de test (3) contenant un complexe (1) selon l'invention contenant des particules d'extraction et au moins une matrice. L'échantillon (2) est ajouté dans le récipient et ceci a pour effet de solubiliser le complexe de l'invention. On obtient donc un récipient contenant l'échantillon mélangé au complexe solubilisé, c'est-à-dire un mélange (4) de l'échantillon avec la matrice solubilisée et les particules d'extraction qui sont désormais libres de leurs mouvements dans la matrice.

En (B), on voit un récipient de test contenant l'échantillon (2). Une portion de complexe (1) est ajoutée dans le récipient et ceci a pour effet de le solubiliser. On obtient donc un récipient contenant l'échantillon mélangé au complexe solubilisé, c'est-à-dire un mélange (4) de l'échantillon avec la matrice solubilisée et les particules d'extraction libres de leurs mouvements dans la matrice.

En (C), on voit un récipient de test (3) contenant un complexe (1) selon l'invention contenant des particules d'extraction et au moins une matrice. Un tampon aqueux (5) ou un tampon de lyse de l'échantillon (5) est ajouté dans le récipient et ceci a pour effet de solubiliser la matrice du complexe. On obtient donc un complexe solubilisé dans le tampon aqueux ou le tampon de lyse c'est-à-dire un mélange (6) comprenant ledit tampon avec la matrice solubilisée du complexe et les particules d'extraction libres de leur mouvement dans la matrice. Ce mélange (6) est transféré dans un récipient comprenant l'échantillon (2). On obtient ainsi un récipient comprenant l'échantillon mélangé au complexe solubilisé dans le tampon aqueux ou le tampon de lyse, c'est-à-dire un mélange (7) de l'échantillon avec le tampon aqueux ou le tampon de lyse, la matrice solubilisée et les particules d'extraction.

En (D), on voit un récipient de test (3) contenant un complexe (1) selon l'invention contenant des particules d'extraction et une ou plusieurs matrices. Un tampon aqueux (5) ou un tampon de lyse de l'échantillon (5) est ajouté dans le récipient et ceci a pour effet de solubiliser la matrice du complexe. On obtient donc la matrice solubilisée dans le tampon aqueux ou le tampon de lyse c'est-à-dire un mélange (6) comprenant ledit tampon avec la matrice solubilisée du complexe et les particules d'extraction libres de leurs mouvements dans la matrice. A ce mélange (6), on ajoute un échantillon (2). On obtient ainsi un récipient comprenant l'échantillon mélangé à la matrice du complexe solubilisée dans le tampon aqueux ou le tampon de lyse, c'est-à-dire un mélange (7) de l'échantillon avec le tampon aqueux ou le tampon de lyse, la matrice solubilisée et les particules d'extraction.

En (E), on voit un récipient de test contenant l'échantillon (2) auquel on ajoute un tampon aqueux (5) ou un tampon de lyse de l'échantillon (5). Ceci a pour effet de lyser l'échantillon. Par ailleurs, on voit un récipient de test (3) contenant un complexe (1) selon l'invention contenant des particules d'extraction et une ou plusieurs matrices. Le lysat obtenu à l'issue de la première étape (mélange de l'échantillon (2) avec le tampon aqueux de lyse (5)) est ajouté au récipient contenant le complexe. Ceci a pour effet de solubiliser la matrice du complexe. On obtient donc le complexe solubilisé dans le lysat et ceci donne le mélange (7) qui correspond à l'échantillon avec le tampon aqueux ou le tampon de lyse, la matrice solubilisée et les particules d'extraction.

En (F), on voit un récipient de test (3) contenant un complexe (1) selon l'invention contenant des particules d'extraction et une ou plusieurs matrices. Un tampon aqueux (5) ou un tampon de lyse de l'échantillon (5) est ajouté dans le récipient et ceci a pour effet de solubiliser la matrice du complexe. On obtient donc le complexe solubilisé dans le tampon aqueux ou le tampon de lyse c'est-à-dire un mélange (6) comprenant ledit tampon avec la matrice solubilisée du complexe et les particules d'extraction. Dans ce mélange (6), on introduit un aimant ou dispositif aimanté (8) qui attire les particules d'extraction magnétiques et ceci d'autant plus lorsque le dispositif aimanté est mis en rotation (symbolisé par la flèche). On retire ce dispositif chargé des particules d'extraction (9). Il est ensuite plongé dans un récipient contenant l'échantillon (2) auquel on a ajouté un tampon aqueux (5) ou tampon de lyse de l'échantillon (5). Le dispositif est à nouveau mis en rotation et ceci a pour effet de remettre en suspension les particules d'extraction dans l'échantillon lysé (lysat). Ces particules d'extraction peuvent ainsi venir capturer les biomolécules de l'échantillon à tester. Dans cette variante de réalisation, on obtient au final un mélange (10) comprenant l'échantillon, le tampon de lyse et les particules. Il n'y a pas un mélange final avec l'échantillon et l'intégralité du complexe solubilisé. Ici, l'échantillon n'est pas mélangé à la matrice solubilisée.

Bien évidemment, cette figure 1 n'est pas exhaustive et il existe de multiples variantes non représentées qui sont couvertes par la présente invention.

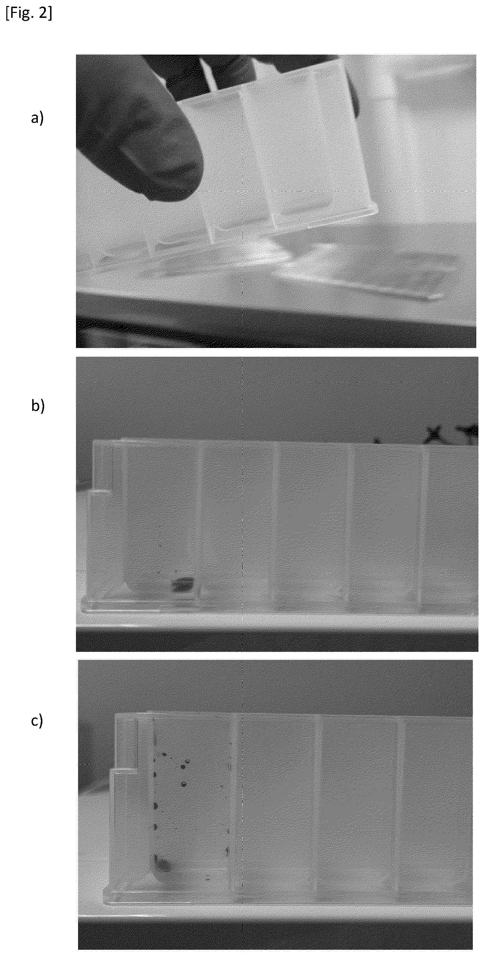

[Fig. 2] est une illustration du problème technique lié aux particules d'extraction qui sont dispersées, projetées sur les parois du puits d'un récipient de test ou dispositif d'extraction des biomolécules lorsque les particules d'extraction ne sont pas immobilisées, maintenues, isolées, compartimentées grâce à un complexe selon l'invention.
a) correspond au récipient de test ou d'analyse biologique en plastique comprenant plusieurs puits.
b) correspond au récipient de test en plastique dans lequel on observe en noir des particules d'extraction (50µl de NucliSENS^{®} easyMAG^{®} MagSIL, bioMérieux) dans le fond d'un des puits du récipient,
c) correspond au récipient de test en plastique dans lequel on observe en noir les particules d'extraction des biomolécules dispersées, projetées sur les parois d'un des puits du récipient de test de l'échantillon, lors du transport et empêchant la récupération totale desdites particules d'extraction et provoquant également leur séchage contre les parois et leur dégradation.

[Fig. 3] correspond un schéma du procédé de fabrication du complexe selon l'invention et du procédé de distribution sur un support ou dans un récipient
A correspond aux particules d'extraction en suspension dans un tampon de stockage, un solvant, un tapon de lyse ou de l'eau, contenues dans un récipient, de type Bécher
B correspond à l'étape où les particules d'extraction sont rassemblées ou maintenues au fond du récipient, soit par centrifugation, soit par aimantation,
C correspond à l'étape où le tampon de stockage des particules d'extraction ou le solvant est retiré , extrait à l'aide ici d'une serigue,
D correspond à l'étape où le tampon de stockage ou le solvant est jeté pour obtenir un récipient ne contenant que les particules d'extraction avec une quantité de liquide (tampon de stockage ou de solvant) minimale de sorte que les particules n'aient pas la possibilité physique et/ou temporelle de sécher et se dégrader,
E correspond à une quantité déterminée de matrice selon l'invention à l'état solide,
F correspond à l'étape de chauffage de la matrice à une termpérature déterminée de sorte qu'elle devienne liquide ou visqueuse (sur cette figure, la température de fusion de la matrice permettant d'obtenir une matrice à l'état liquide est comprise entre 50 et 60°C),
G correspond à une représentation de la matrice à l'état liquide ou visqueux,
H correspond à l'étape de mise en contact de la matrice à l'état liquide ou visqueux avec les particules d'extraction obtenues à l'issue de l'étape D, de sorte à obtenir un mélange ou une suspension avec des particules d'extraction à une concentration donnée dans la matrice,
I correspond à une représentation de ce mélange ou suspension obtenue à l'issue de l'étape H,
J correspond à une étape d'agitation ou de mélange de la suspension comprenant les particules d'extraction et la matrice à l'état liquide, de sorte à obtenir un mélange ou une suspension homogène formant ainsi le complexe selon l'invention à l'état liquide,
A Bis correspond à l'étape où il y a une réitération de l'étape B et des suivantes, ceci dans le but de rincer les particules d'extraction et de retirer au maximum toute trace du tampon de stockage, du solvant, du tampon de lyse ou l'eau dans lequel les particules d'extraction étaient contenues initialement. Ceci vise à limiter la présence d'une phase aqueuse pour ne pas solubiliser la matrice qui est hydrosoluble et lui permettre de solidifier en refroidissant et de former ainsi le complexe selon l'invention à l'état solide,
K correspond à l'étape de distribution de la suspension homogène obtenue à l'issue de l'étape J, ceci se fait comme dans la figure 4 ou 5.

[Fig. 4] correspond à une variante du procédé de distribution du complexe selon l'invention et une illustration du complexe selon l'invention dans lequel la matrice est dans un état solubilisé.
A correspond à une illustration du complexe selon l'invention à l'état liquide, les particules d'extraction sont mobiles et libres de leur mouvement dans la matrice hydrosoluble qui est à l'état liquide à une temperature supérieure au point de fusion de la matrice. Le complexe est contenu dans un récipient de type Bécher pour permettre de verser ou couler le complexe à l'état liquide en maitenant une température appropriée dans un ou plusieurs récipients adaptés, par exemple de type récipient de test biologique.
B correspond au complexe à l'état liquide distribué dans trois récipients, ce nombre n'étant qu'illustratif,
C correspond à l'étape de refroidissement naturel ou provoqué/accéléré du complexe selon l'invention, de sorte qu'on obtienne un complexe selon l'invention à l'état solide comme ceci est représenté
en D où le complexe est non seulement solide mais il adhère à la paroi du récipient et ne tombe pas lors du retournement de celui-ci. Les particules d'extraction sont donc bien dispersées dans la matrice solide, elles y sont donc piégées et ne peuvent pas se disperser, se disséminer sur les parois du récipient ou support. La matrice reste solide et maintient, immobilise ou compartimente les particules d'extraction dans le fond du récipient tant que la température de cette matrice reste en -dessous de son point de fusion et tant que la matrice n'est pas mise en contact d'une composition aqueuse (par exemple, tampon de stockage, tampon de lyse, eau ou échantillon à tester).
E correspond à l'étape d'ajout d'une composition aqueuse (par exemple le tampon de lyse) sur le complexe à l'état solide.
F correspond à l'illustration du résultat de cet ajout de la composition aqueuse (par exemple le tampon de lyse) au complexe solide selon l'invention. Ceci a donc pour effet de solubiliser la matrice du complexe et de libèrer les particules d'extraction qui sont ainsi libres de leurs mouvement dans la matrice solubilisée. Elles peuvent ainsi capturer les biomolécules de l'échantillon à tester lorsque celui-ci sera ajouté au mélange tampon de lyse-complexe solubilisé.

[Fig. 5] correspond à des variantes du procédé de distribution du complexe selon l'invention.

Avant que le complexe ne soit dans son état solide, lors de son refroidissement ou lorsque le complexe dans son état solide reste suffisamment visqueux, pâteux ou malléable, en fonction de sa composition, la distribution du complexe selon l'invention peut se faire
- soit à l'aide d'un ustensile adapté tel qu'une seringue comme on le voit en L où la seringue dépose le complexe à l'état liquide ou visqueux au fond du récipient de test biologique,
- soit, comme on le voit en M, à l'aide d'une spatule où le complexe à l'état solide est déposé sur un support ou un récipient de test.

N correspond à un complexe selon l'invention à l'état solide après refroidissement (naturel ou artificiel) distribué selon le procédé décrit à la figure 4. On voit qu'il forme un culot de volume plein, sans volume intérieur, c'est-à-dire sans vide intérieur, et qu'il ne tapisse pas uniquement les parois du récipient. Il adhère aux parois et entre chacunes d'elles forme une structure continue.

[Fig. 6] correspond à une illustration du complexe (A) selon l'invention comparée à des solutions déjà existantes (B, C et D)
A correspond au complexe selon l'invention dans un récipient. On peut voir l'axe longitudinal Y-Y' du récipient et la hauteur déterminée du complexe selon cet axe ainsi que l'axe transversal du récipient et la largeur déterminée du complexe selon cet axe dans le récipient. On observe bien que, dans ce récipient, le complexe épouse les parois du récipient qui le contient, a un volume plein et ne présente pas de volume intérieur ou volume vide (VV)au centre du récipient comme ceci est observé en B.
B correspond à une solution existante mais ne donnant pas satisfaction. Il s'agit d'un film contenant des particules d'extraction, séché puis poussé au fond du récipient. Ce film est plaqué contre les parois du récipient laissant ainsi un volume vide (VV) selon les axes X-X' et Y-Y' du récipient, un espace central. Cette solution n'est pas satisfaisante pour plusieurs raisons :
   - il est difficile que le film épouse parfaitement les parois du récipient et reste en place lors du transport du récipient. Si le film se décolle du fond du récipient, il peut se retrouver le long des parois ou au niveau de l'opercule du récipient et engendrer les problèmes de dispersion/dissémination des particules et de non-fiabilité du test.
   - Ce film étant séché lors de sa production, les fonctionnalités et performances des particules d'extraction sont altérées. La fine épaisseur du film augmente également ce séchage et l'altération des performance desdites particules d'extraction.
   - De plus, la mise en œuvre d'une telle solution est beaucoup plus compliquée car nécessite la formation préalable d'un film, nécessite plus de matériel, plus coûteuse, plus longue et moins reproductible que la solution proposée par l'invention.
C correspond à un récipient contenant des particules d'extraction (1) dans le fond du récipient, lesquelles sont recouvertes d'un film (2), de préférence de matière plastique pour les maintenir à une localisation donnée (le fond du récipient) et éviter leur dispersion lors du transport du récipient. Cependant, C ne donne pas totalement satisfaction car il est difficile de maintenir le film en place sans qu'il y ait de fuite, ce qui engendre la dispersion des particules lors du transport du récipient et tous les problèmes évoqués plus haut. De plus, cette solution est difficile à mettre en œuvre de façon automatisée. Lors des étapes mises en œuvre pour l'extraction et la détection des biomolécules, le film plastique risque également de gêner les différentes étapes, en particulier il risque de gêner les pipetages ainsi de fausser les résultats d'analyse.
D correspond à un aimant (3) recouvert des particules d'extraction (1) qui sont magnétiques. L'aimant permet ainsi de maintenir les particules d'extraction à une localisation donnée du récipient et empêche ainsi leur dispersion. Cette solution n'est pas totalement satisfaisante car, d'une part, les particules d'extraction pourraient sécher et être altérées dans leurs performances ultérieures, d'autre part, l'aimant de reste pas nécessairement en place lors du transport du récipient mais surtout il est difficile d'effectuer les étapes de captures d'extraction et détection des biomolécules avec un aimant dans le récipient. Ceci entraine des interférences et une reproductibilité du test non satisfaisante. Une autre solution avec un aimant extérieur au récipient a également été envisagée. Cette solution est satisfaisante pour les étapes d'extraction et de détection des biomolécules mais ceci est beaucoup plus difficile à mettre en œuvre, est plus coûteux et/ou donne des résultats non-satisfaisants lors du transport du récipient, ce que la présente invention solutionne parfaitement et simplement.

[Fig. 7] correspond à des représentations du complexe selon l'invention sous forme solide dans un récipient.
On voit deux récipients (de type Falcon) contenant un complexe selon l'invention sous forme solide et adhérant au fond du récipient en formant un culot adhésif.
Ce complexe est constitué de 50 µl de particules de silice magnétiques à 20mg/ml dans une matrice d'agarose à 2% (A) ou de gélatine à 2% (B).

### EXEMPLES

### Exemple 1 - Protocole d'extraction générique et d'analyse par PCR des acides nucléiques d'un échantillon biologique

Afin d'évaluer la compatibilité des différentes matrices avec le maintien des performances d'extraction des particules de silice magnétiques (particules d'extraction), le protocole suivant est mis en œuvre. Il utilise différentes souches bactériennes aliquotées dans différents types d'échantillons biologiques comme indiqué ci-dessous.

### Echantillons fécaux

On dispose des souches mères de microorganismes modèles qui peuvent être utilisées en mélanges: *Salmonella enterica* (SLM), *Yersinia enterocolitica* (YER) issues de souches ATCC (ATCC 9263 pour SLM et ATCC 55075 pour YER), *Adenovirus 40* (ADV), de Entero Virus coxsakievirus strain 2 (Entero) (Zeptometrix, ref 0810075CF), et de *Rotavirus* (ROTA) issus d'une source interne (bioMérieux, Verniolle, France).

On ajoute à un milieu de transport liquide (Fecal swab COPAN, référence 470CE.A) les micro-organismes vivants ci-dessus pour obtenir des concentrations respectives de 1,4x10e6 cp/mL en SLM, de 2,5x10e6 cp/mL en YER, de 1,6x10e6 cp/mL en ADV, de 110 TCID50/ml en Entero ou de 2,1e7 cp/mL en ROTA. Un prélèvement de fèces est ensuite réalisé avec l'écouvillon qui est transféré dans le milieu de transport liquide ensemencé dans lequel il est agité par vortex.

### Echantillons respiratoires (RESPI)

On dispose de la souche mère du microorganisme modèle Legionella (LEG), (Zeptometrix, ref: 0801645) qui est ajoutée à l'échantillon respiratoire pour obtenir une concentration de 5,9x10e4 cp/mL en LEG.

### Echantillons urinaires

On dispose des souches mères de microorganismes modèles: Chlamydia trachomatis (CT) (Zeptometrix, ref : 0804390) et Neisseria gonorrhoeae (NG) (Zeptometrix, ref : 0801482). De la même manière, ces bactéries sont ajoutées à l'échantillon urinaire afin d'obtenir une concentration respective de 1,0x10e2 cp/mL en CT et de 1,1x10e2 cp/mL en NG.

Les échantillons préparés ci-dessus sont ensuite traités sur une plateforme automatisée d'extraction des acides nucléiques eMAG^{®} (BioMérieux, Marcy l'Etoile, France) équipée des réactifs d'extraction NucliSENS^{®} (Tampon de lyse, tampons de lavage et tampon d'élution, billes de silice magnétiques...) en suivant le protocole du fournisseur (BioMérieux 50). Soit brièvement:
200µL des échantillons biologiques respiratoires, fécaux ou urinaires ensemencés ci-dessus sont ajoutés à 2ml de tampon de lyse NucliSENS^{®} eMAG^{®} Lysis Buffer auquel on ajoute 50µl de la suspension de particules magnétiques commerciale à 20 mg/ml (MagSIL NucliSENS^{®} easyMAG^{®} Magnetic Silica ref 280133, bioMérieux, Marcy l'Etoile, France) en tant que référence (REF - condition témoin).

La préparation est traitée successivement par les tampons de lavage et d'élution en même temps que des cycles d'aimantation sont réalisés par l'automate.

L'éluât obtenu après extraction contenant les acides nucléiques des différents microorganismes est ensuite mélangé à différents premix PCR contenant le nécessaire pour amplifier les acides nucléiques correspondants. Ces premix proviennent respectivement du kit LEGIO PNEUMO/CC R-GENE^{®} 71-046 pour la détection de LEG et de formulations PCR ad hoc avec pour chaque réaction 0.4µM de primers, 0.2µM de sonde TaqMan^{®} et 0.4U/µL de Taq polymerase (et pour ROTA (Reverse Transcriptase) 1U/µL) pour la détection de SLM, ADV, YER, CT et NG respectivement. Chaque amplification de LEG a été faite selon les recommandations du kit LEGIO PNEUMO/CC R-GENE^{®} pour la détection de LEG. Pour les autres microorganismes, on a utilisé les conditions de PCR suivantes : 7,5 µl de premix mélangés à 7,5µl d'éluât avec une étape de transcription inverse - reverse transcription (quand nécessaire) pendant 5 minutes à 55°C pour un cycle, une étape d'activation de la Taq polymerase pendant 2 minutes à 95°C pour un cycle, une étape de dénaturation pendant 3 secondes à 95°C et une étape d'hybridation pendant 35 secondes à 60°C pour 45 cycles (dénaturation et hybridation). La détection de la PCR se fait par la technologie TaqMan^{®} sur un thermocycler CFX 96 (BioRad, Hercules, USA) selon le protocole recommandé par le fournisseur afin de collecter les valeurs de Ct (Cycle Threshold) caractéristiques des performances de l'étape d'extraction avec la silice magnétique. Ces Ct permettront ainsi d'évaluer les performances de l'étape d'extraction et ainsi des particules d'extraction en fonction des différentes conditions : les conditions témoins et les conditions selon l'invention dans laquelle les particules d'extraction se trouvent incluses dans une matrice donnée (différents constituants). Les résultats sont indiqués dans les exemples ci-après.

### Exemple 2 - Comparaison entre l'extraction d'acides nucléiques réalisée avec des particules d'extraction séchées et non séchées.

Un échantillon de fèces est extrait et amplifié selon le protocole donné dans l'exemple 1 en présence, d'un côté, de particules commerciales (MagSIL NucliSENS^{®} easyMAG^{®} Magnetic Silica ref 280133, bioMérieux, Marcy l'Etoile, France - notées Particules magnétiques REF LIQUIDE dans le tableau) en suspension liquide dans le tampon aqueux de stockage dans lequel elles sont vendues et prêtes à l'emploi et, de l'autre côté, avec la même quantité des mêmes particules commerciales mais séchées préalablement par évaporation à l'air libre et à température ambiante (notées Particules magnétiques SECHEES dans le tableau 1).

Le tableau 1 ci-dessous montre les performances observées sur l'extraction SLM, YER et ADV dans chaque condition (particules d'extraction en milieu liquide et particules d'extraction séchées). Le contrôle d'amplification n'a pas montré de différence, il n'y a pas eu d'inhibition de la PCR pour chacune des amplifications (non montré). Le Ct (Cycle Threshold ou cycle seuil) correspond au nombre de cycles nécessaires pour que la fluorescence générée par l'amplification du produit PCR dépasse un niveau de seuil détectable. La valeur Ct est inversement proportionnelle à la quantité initiale de la cible dans l'échantillon : plus la valeur Ct est basse, plus la quantité initiale de la cible dans l'échantillon est élevée.

Lorsque les particules d'extraction sont séchées, on observe un retard de Ct sur les 3 tests PCR (Moyenne Ct SLM, YER, ADV) par rapport à la référence liquide. Puisqu'il n'y a pas d'inhibition de la PCR, le retard est nécessairement dû à la quantité d'acides nucléiques initiale contenue dans l'éluât et ceci reflète donc les performances d'extraction des particules d'extraction. Ce retard de Ct (calculé par la différence entre le Ct des particules séchées et le Ct de la référence liquide - cette différence est notée Delta Ct (Séchées-Ref. liquide)) est égal ou supérieur à 1,5 et montre que l'extraction a été moins efficace avec les particules séchées (presque 10 fois moins d'ADN extraits pour YER par exemple).

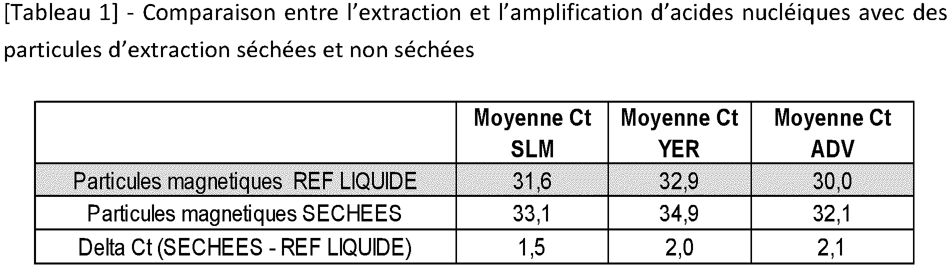

On comprend donc que le séchage des particules d'extraction de silice magnétique impacte les performances d'extraction des acides nucléiques par les particules d'extraction. Par conséquent, les particules d'extraction ne doivent pas être sèches ou séchées si l'on veut conserver une bonne sensibilité et rapidité du test PCR. Il faut donc trouver une solution pour que ces particules ne soient pas dispersées sur les parois du récipient de test, désolidarisées et séchées.

### Exemple 3 - Procédé de fabrication et de distribution d'un complexe selon l'invention comprenant différentes matrices

### Exemple 3a - Procédé de fabrication et de distribution d'un complexe selon l'invention comprenant des particules d'extraction de biomolécules (acides nucléiques) incluses dans une matrice constituée d'un mono alcool - Immobilisation des particules d'extraction après solidification de la matrice

Une suspension de 1 ml de particules magnétiques (MagSIL NucliSENS^{®} easyMAG^{®} Magnetic Silica ref 280133, bioMérieux, Marcy l'Etoile, France) à 20 mg/ml est centrifugée 3 min avec une mini centrifugeuse (2000g pendant 5 min) de paillasse, puis le surnageant est éliminé par pipetage (environ 1 ml). Il est remplacé par un volume équivalent de matrice selon l'invention, ici le Tert-butanol pur (Sigma, ref : 360538) ou le 2,2 diméthyl propanol pur (Sigma, N7206), préalablement chauffé et ajouté à une température de l'ordre de 60 à 85°C, qui est une température au-delà de la température de fusion (point de fusion) de la matrice de sorte que la matrice soit sous forme liquide. La centrifugation, l'élimination du surnageant et le rajout d'un volume d'alcool (tert-butanol ou 2,2 diméthyl propanol) sont répétés jusqu'à deux fois afin d'éliminer et de remplacer un maximum de la solution aqueuse de la suspension initiale de particules magnétiques. Il est donc primordial que la matrice soit hydrosoluble pour permettre de retirer au maximum la phase aqueuse qui contenait initialement les particules d'extraction. On obtient une suspension finale de particules d'extraction à 20 mg/ml dans la matrice désirée (Tert-butanol ou 2,2 diméthyl propanol) que l'on peut distribuer à chaud dans les récipients adaptés. Le mélange va donc être versé dans les récipients et va épouser la forme du récipient dans lequel il est distribué (par coulage ou versage). On laisse la matrice contenant les particules magnétiques refroidir à température ambiante voire en abaissant la température aux alentours de 4°C. Ainsi, en refroidissant le mélange passe de l'état liquide à un état solide ou pâteux de sorte à obtenir un complexe selon l'invention qui adhère au récipient dans lequel il est moulé. C'est donc un complexe qui comprend une matrice solide, figée et qui contient ou piège les particules d'extraction. La formation dudit complexe se fait sans étape de séchage, ce qui aurait eu pour conséquence de dégrader les performances de particules du complexe comme vu dans l'exemple précédent. Dans le présent exemple, on a distribué une suspension de 50µl à 20mg/ml, soit 1mg de particules d'extraction.

### Exemple 3b - Procédé de fabrication et de distribution d'un complexe selon l'invention comprenant des particules d'extraction dans une matrice lipidique selon l'invention - Immobilisation des particules d'extraction après solidification de la matrice.

Le remplacement du tampon aqueux de resuspension ou de stockage des particules magnétiques est effectué comme dans l'exemple 3a à la différence qu'aucun alcool n'est utilisé pour le lavage des particules. En lieu de l'alcool, on utilise une matrice lipidique chauffée également à une température comprise entre 50°C et 85°C de sorte qu'elle soit sous forme liquide : le LABRAFAC^{®} MC60, le GELOIL^{™} SC ou le LABRASOL^{™} ALF (Gattefossé, Saint Priest, France) pour éliminer le tampon aqueux. À la suite de ce lavage, les particules sont reprises dans un volume équivalent au volume retiré dans une matrice lipidique finale qui est également chauffée pour en faciliter la distribution. La matrice finale est le LABRAFAC^{®} MC60 pur, le GELOIL^{™} pur ou un mélange de LABRAFAC MC60 et de GELUCIRE^{®} 48/16 (Gattefossé, Saint Priest, France) dans un ratio de 85/15, 50/50, 40/60 ou 20/80. Le mélange est distribué à chaud dans un récipient de sorte qu'il épouse la forme du récipient et donne une fois refroidi un complexe moulé. Dans une variante de cet exemple, le mélange ou complexe est laissé à refroidir en l'état. Il est ensuite fractionné ou partitionné de sorte que l'on ait l'équivalent de 1mg de particules d'extraction dans chaque fraction qui est ensuite distribuée sous forme de pâte à l'aide d'une seringue dans le fond d'un récipient d'analyse biologique.

### Exemple 3c - Procédé de fabrication et de distribution d'un complexe selon l'invention comprenant des particules d'extraction dans une matrice constituée d'un polymère hydrosoluble (gélatine ou agarose selon l'invention) - Immobilisation des particules d'extraction après solidification de la matrice.

Une suspension de 1 ml de particules magnétiques (MagSIL NucliSENS^{®} easyMAG^{®} Magnetic Silica ref 280133, bioMérieux, Marcy l'Etoile, France) à 20 mg/ml est centrifugée pendant 3 min avec une minicentrifugeuse de paillasse (2000g pendant 5 min), puis le surnageant est éliminé par pipetage (environ 1 ml). Il est remplacé par un volume équivalent (environ 1ml) de matrice selon l'invention, ici de la gélatine à 2% ou de l'agarose à 2% dans du MES pH 5.5 préalablement chauffé et ajouté à une température de l'ordre de 85°C pour chaque condition, au-delà du point de fusion respectif de ces composés de sorte que la matrice soit sous forme liquide. On obtient une suspension finale de particules d'extraction à 20 mg/ml dans la matrice désirée que l'on peut distribuer en versant à chaud par aliquots de 50 µl (soit 1 mg de particules) dans les récipients adaptés (mini tube en polypropylène de 1ml, mini-tube type Eppendorf de 1ml). On laisse la matrice contenant les particules magnétiques refroidir et se solidifier à température ambiante, de sorte qu'elle forme un complexe moulé dans ce récipient.

### Exemple 4 - Choix des matrices du complexe pour l'immobilisation des particules d'extraction et performances des particules d'extraction

Un échantillon biologique est extrait et amplifié selon le protocole donné dans l'exemple 1 en présence, d'un côté, de particules commerciales (MagSIL NucliSENS^{®} easyMAG^{®} Magnetic Silica ref 280133, bioMérieux, Marcy l'Etoile, France - notées Particules magnétiques de référence - REF LIQUIDE (1 ou 2) dans le tableau 2 ci-dessous) en suspension liquide dans le tampon aqueux de stockage des particules d'extraction et, de l'autre côté, avec la même quantité des mêmes particules reprises dans une matrice selon l'invention, comme préparé dans l'exemple 3a (matrice mono-alcool), dans l'exemple 3b (matrice lipidique) ou dans l'exemple 3c (matrice de polymères hydrosolubles). La matrice était dans son état solide lors des tests effectués pour cet exemple.

Ainsi, nous avons pu évaluer les différents complexes selon l'invention et par conséquent l'effet des matrices sur la performance des particules d'extraction pour l'extraction des acides nucléiques et l'efficacité de la PCR. Un comparatif est réalisé avec chaque matrice par rapport à un témoin qui est représenté par les particules de silice magnétique de référence seule (REF liquide).

Ce tableau 2 permet de calculer ensuite une différence de Ct (noté Delta Ct) entre les manipulations réalisées avec les particules de silice magnétique incluses dans une matrice donnée (invention) et les particules de silice magnétique de référence seule. Un delta Ct +1,5 (retard de 1,5 Ct par rapport à la référence) est considéré comme impactant et non acceptable. Un delta Ct inférieur à +1,5 est considéré comme n'impactant pas l'extraction des acides nucléiques de l'échantillon, ni la PCR. On comprend qu'un delta Ct négatif (inférieur à 0) est considéré comme significatif d'une amélioration de l'extraction des acides nucléiques.

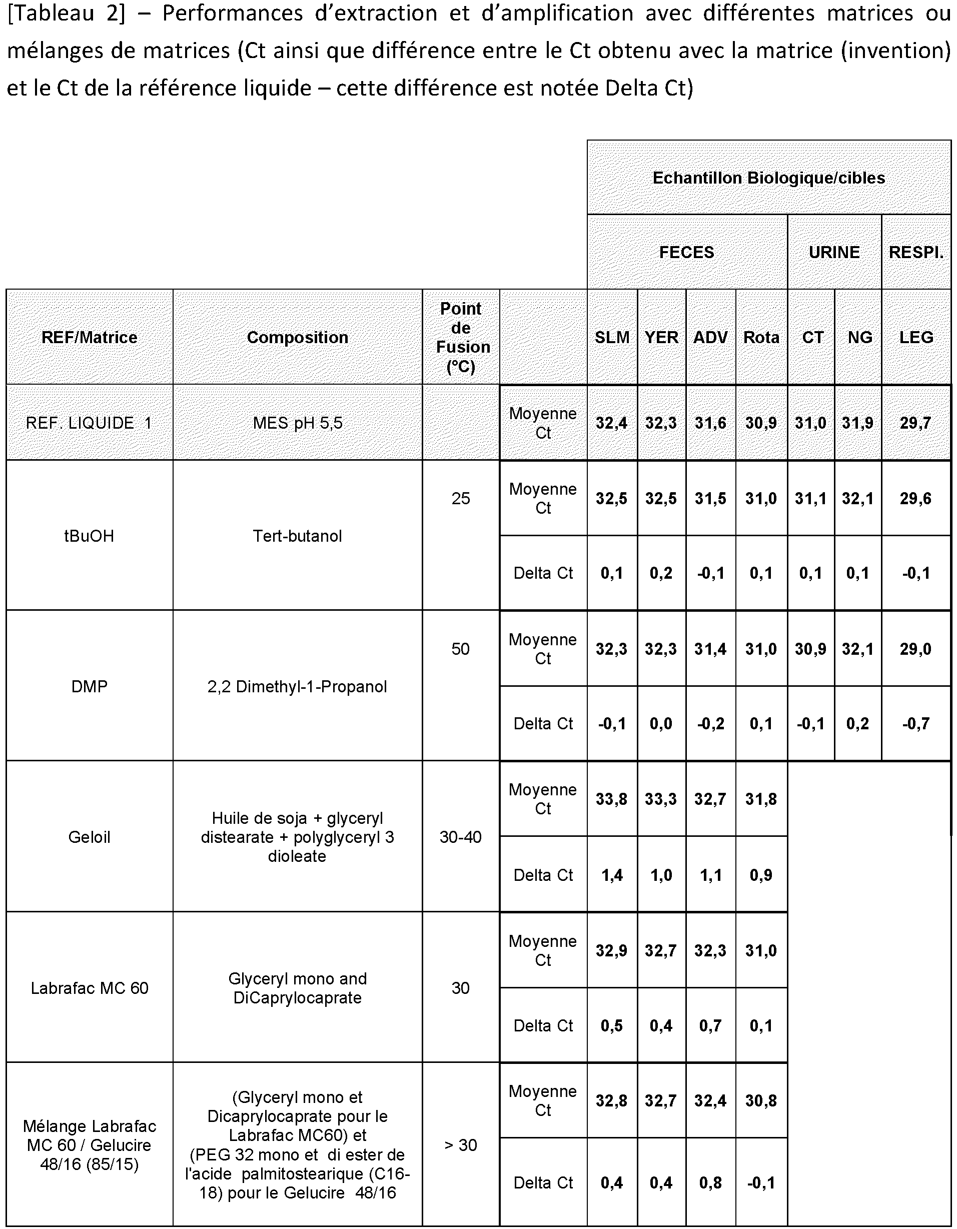

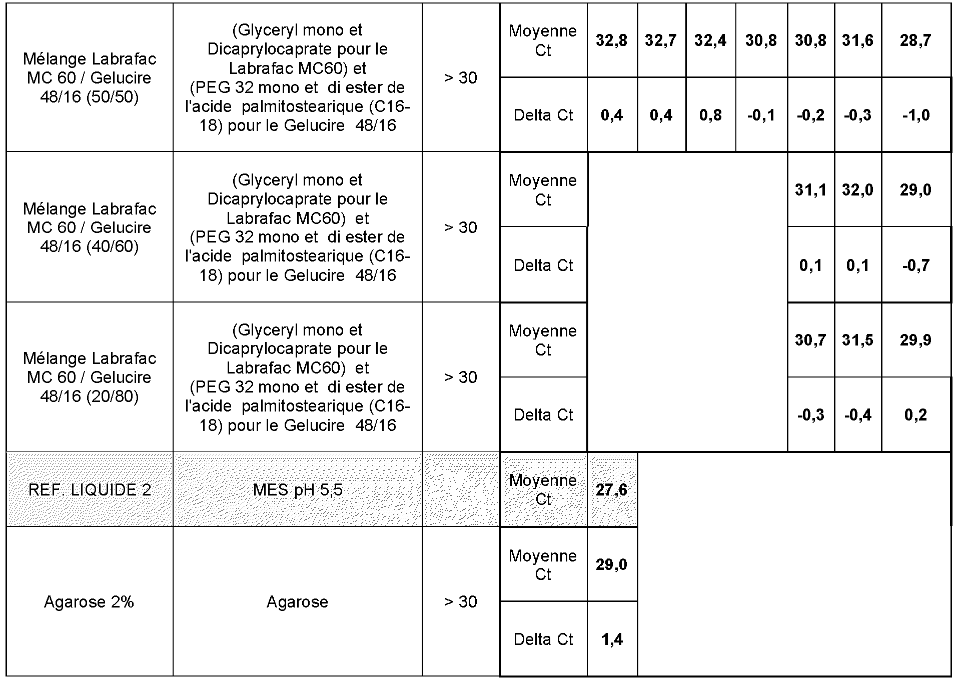

Il est démontré ici sur le Tableau 2 que les matrices des complexes sélectionnées et testées ne causent pas une baisse significative des performances des particules magnétiques pour l'extraction des acides nucléiques (différence de Ct +/- 1 ou du moins en-dessous de 1,5 pour tous les complexes selon l'invention) et ce quel que soit le type d'échantillon biologique dans lequel les microorganismes et donc les acides nucléiques se trouvent. Dans le cas du DMP (2,2-DiMéthyl-1-Propanol), il est même observé une amélioration des performances d'extraction des acides nucléiques pour SLM, YER, ADV et LEG (différence de Ct de 0.1). Le contrôle d'amplification n'a pas montré de différence, il n'y a pas eu d'inhibition de la PCR pour chacune des amplifications.

### Exemple 5 : Stabilité des particules magnétiques contenues dans une matrice solidifiée de 2,2 dimethyl-1-propanol et démonstration de la conservation des performances d'extraction et d'amplification

Des échantillons biologiques sont extraits et amplifiés selon le protocole donné dans l'exemple 1 en présence, d'un côté, de particules commerciales (MagSIL NucliSENS^{®} easyMAG^{®} Magnetic Silica ref 280133, bioMérieux, Marcy l'Etoile, France - notées Particules magnétiques de référence - REF LIQUIDE dans le tableau 3) en suspension liquide dans le tampon aqueux de stockage des particules d'extraction et, de l'autre côté, avec la même quantité des mêmes particules reprises dans une matrice choisie comme préparé dans l'exemple 3a dans le cas de matrices d'alcools, en l'occurrence le 2, 2-diméthyl -1- propanol. Les performances d'extraction et d'efficacité de la PCR sont mesurées une fois par mois sur une durée de 3 mois afin de démontrer la stabilité du complexe selon l'invention et surtout des particules d'extraction stockées dans la matrice solide (mesure des Ct et de la différence de Ct par rapport à la référence liquide comme décrit dans l'exemple 4 - cette différence est notée Delta CtT0 ou T3mois). La température de stockage est de 4°C.

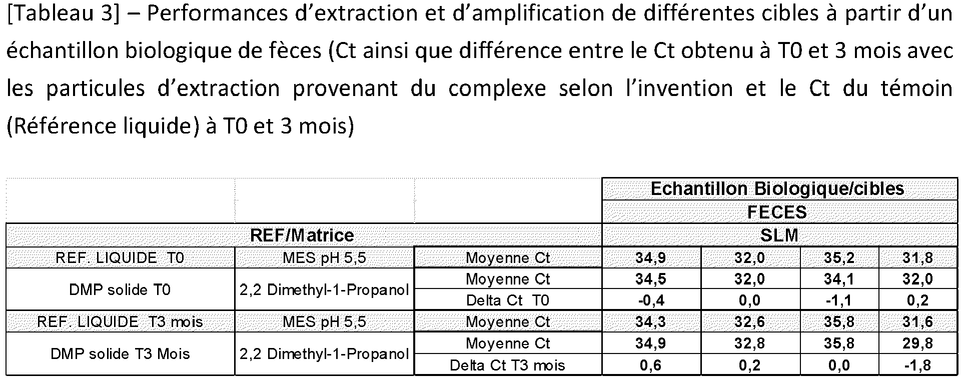

Il est ainsi démontré dans le Tableau 3 que les particules d'extraction conservées dans un complexe avec une matrice sous forme solide (mais pas liquide) sont aussi performantes que les particules stockées en milieu liquide.

On constate que le procédé de fabrication du complexe selon l'invention par refroidissement (naturel ou forcé) de la matrice menant à sa solidification et renfermant ainsi les particules d'extraction (silice magnétique) donne des complexes selon l'invention stables plusieurs mois et dans lesquels la matrice n'affecte ni la stabilité, ni l'intégrité et ni la fonctionnalité des particules d'extraction.

Ainsi, il sera bien compris de ce qui précède, des exemples donnés et des figures qu'il est possible d'adapter le type de matrice ou de mélange de matrices afin d'obtenir les performances d'extraction attendues. Les matrices décrites sont donc d'excellents candidats pour entrer dans la composition des complexes selon l'invention. Sous forme refroidie et donc solide, elles permettent ainsi de piéger, immobilliser ou contenir les particules d'extraction (silice magnétique) dans un lieu précis et ainsi de former un complexe solide n'ayant pas ou très peu d'impact sur l'extraction des acides nucléiques. Ainsi, lors de la mise en contact du complexe avec de l'eau, un tampon aqueux, un tampon de lyse et/ou l'échantillon, la matrice du complexe va se solubiliser, libérant ainsi les particules d'extraction qui ne seront alors plus piégées, figées dans la matrice mais libres de leurs mouvements et qui vont alors pouvoir entrer en contact avec les biomolécules provenant de l'échantillon à tester et les capturer. Ces différentes matrices sont compatibles avec les performances des particules d'extraction tout en empêchant les particules de sécher et de se disperser dans tout le consommable plastique, le récipient d'analyse biologique ou le support comprenant le complexe.

Grâce au complexe, la manipulation des particules d'extraction est facilitée. Ceci permet également d'avoir un dosage précis des particules magnétiques mises en contact avec l'échantillon et d'optimiser le rendement de capture.

Le complexe selon l'invention est facilement manipulable ce qui simplifie la préparation du récipient où le test sera effectué, le récipient destiné à accueillir l'échantillon à tester. Le complexe selon l'invention, qu'il soit sous forme liquide ou une fois refroidi et donc sous forme solide, peut ainsi être distribué aisément dans un puits du récipient de test. Il apporte donc une solution au problème de distribution des particules d'extraction dans le récipient, cette distribution pouvant aisément être automatisée et, par sa propriété de confinement ou compartimentation des particules d'extraction, il apporte également une solution au problème de dissémination de ces dernières sur les parois et/ou le couvercle du récipient lors du transport ou la manipulation de celui-ci et évite ainsi la diminution de leurs propriétés de capture (performance car séchage) mais aussi la diminution du nombre de particules pouvant capturer des biomolécules. Il permet également de contenir les particules d'extraction dans une matrice protectrice, ce qui a pour effet de maintenir leur stabilité mais aussi d'empêcher leur séchage qui dégrade leurs performances. Le complexe selon l'invention contribue in fine à obtenir une extraction des biomolécules propre, efficace, optimale, fiable, rapide et reproductible.

Ainsi, tous les problèmes techniques rencontrés et évoqués plus haut sont solutionnés par le complexe selon la présente l'invention et les procédés le mettant en œuvre et ceci grâce à une sélection des constituants de la matrice permettant aux particules d'extraction de conserver toutes leurs propriétés lors du stockage mais aussi lors d'un procédé d'extraction ou de détection des biomolécules d'un échantillon à tester.

Bien entendu, l'invention n'est pas limitée aux modes de réalisation décrits et représentés dans les figures annexées. Des modifications restent possibles, notamment du point de vue de la constitution des divers éléments ou par substitution d'équivalents techniques, sans sortir pour autant du domaine de protection de l'invention.

## Revendications

1. Complexe comprenant au moins :
- une matrice soluble dans un tampon aqueux, dans un tampon de lyse des cellules d'un échantillon à tester et/ou dans l'échantillon liquide à tester,
- des particules d'extraction de biomolécules, de préférence des particules de silice et plus préférentiellement des particules de silice magnétiques,
ledit complexe est configuré pour se présenter sous au moins deux états distincts :
- un état solide où la matrice est solide et les particules d'extraction de biomolécules sont incluses et non-mobiles dans la matrice, et
- un état liquide où la matrice est liquide ou solubilisée et les particules d'extraction sont libres de leurs mouvements dans ladite matrice.

2. Complexe selon la revendication précédente, dans lequel la matrice est :
• solide à une température située en-dessous d'une première température déterminée,
• liquide entre cette première température et 200°C, et
• solubilisée lorsqu'elle est en contact d'un tampon aqueux, d'un tampon de lyse des cellules d'un échantillon à tester et/ou d'un échantillon liquide à tester.

3. Complexe selon l'une des revendications précédentes, dans lequel la matrice a un point de fusion compris entre 20°C et 85°C.

4. Complexe selon l'une des revendications précédentes, **caractérisé en ce que** le complexe, dans son état solide, a une viscosité supérieure ou égale à 10 Pa.s.

5. Complexe selon l'une des revendications précédentes, dans lequel la matrice comprend au moins un mono-alcool, de préférence ayant moins de 9 atomes de carbone, tel que le tert-butanol, le diméthyl propanol, le cyclohexanol substitué ou non; de la gélatine en solution aqueuse, de préférence de la gélatine de bœuf, de porc ou de poisson; de l'agar-agar en solution aqueuse ou ses dérivés tels que l'agarose; au moins un acide carboxylique en C6 à C20 estérifié par un alcool ou par un alcool polyéthoxylé, tel que du PEG stéarate; du glycérol éthérifié totalement ou partiellement par une chaîne polycarbonée en C6 à C20 et/ou par une chaîne polyéthoxylée; du glycérol estérifié totalement ou partiellement par un ou des acides carboxyliques à chaine en C6 à C20 et/ou polyéthoxylée, tel que du glycéryl monocaprylocaprate, glycéryl dicaprylocaprate ou un mélange des deux; du glycérol éthérifié et estérifié totalement ou partiellement par une chaîne polycarbonée en C6 à C20 et/ou polyéthoxylée, tel que du caprylocaproyl polyoxyl-8 glycéride ; un oligomère ou un polymère du glycérol estérifié totalement ou partiellement par des acides carboxyliques à chaîne en C6 à C20 et/ou polyéthoxylés, tel que du polyglycéryl-3 dioléate ; un dérivé du propylène glycol estérifié par un acide carboxylique à chaîne polycarbonée en C6 à C20 et/ou polyéthoxylée, tel que du propylène glycol caprylate ; ou un mélange d'au moins deux d'entre eux, avec ou sans huile végétale, comme l'huile de soja ou l'huile d'amande.

6. Complexe selon l'une des revendications précédentes, dans lequel la matrice est composée de tert-butanol ou d'un isomère du pentanol, de préférence le 2,2 -diméthyl - 1- propanol, ou de mono et dicaprylocaprate de glycéryle en mélange avec un ester entre l'acide palmitique et/ou l'acide stéarique et un alcool polyéthoxylé, de préférence un PEG (n=32).

7. Complexe selon l'une des revendications précédentes, **caractérisé en ce qu'**il est destiné à être coulé ou versé à l'état liquide dans un récipient à une température comprise entre 20°C et 130°C en fonction de la matrice du complexe.

8. Procédé de fabrication d'un complexe selon l'une des revendications précédentes comprenant les étapes suivantes:
a) Prendre des particules d'extraction de biomolécules d'un échantillon à tester,
b) Lorsqu'il est présent, retirer le liquide dans lequel les particules d'extraction de biomolécules d'un échantillon à tester sont contenues, ledit liquide pouvant être un tampon de stockage, un solvant, un tampon aqueux, de l'eau ou une matrice au moins partiellement solubilisée,
c) Mettre en contact lesdites particules d'extraction de biomolécules avec une matrice telle que définie dans les revendications précédentes à une température à laquelle ladite matrice est liquide pour former un mélange ou suspension de particules d'extraction des biomolécules à une concentration donnée dans la matrice liquide,
c') Optionnellement, réitérer l'étape b) et c),
d) Refroidir ou laisser refroidir naturellement le mélange ou suspension en vue d'obtenir le complexe sous forme solide.

9. Procédé de distribution sur un support ou dans un récipient du complexe selon l'une des revendications 1 à 7 ou du complexe produit selon la revendication 8, comprenant au moins une des étapes suivantes :
A) Lorsque le complexe est à l'état liquide, le couler, le verser ou le dispenser à l'aide d'un ustensile adapté sur un support ou dans un récipient, de préférence un récipient d'analyse biologique,
B) Refroidir ou laisser refroidir naturellement le complexe distribué de sorte à ce qu'une fois à l'état solide le complexe ait un volume plein, dépourvu de volume intérieur ou qu'il se présente sous la forme d'un culot,
ou
C) Lorsque le complexe est sous forme solide, déposer au moins une fraction dudit complexe sur le support ou dans le fond du récipient de sorte qu'il forme un complexe ayant un volume plein, dépourvu de volume intérieur.

10. Procédé d'immobilisation de particules d'extraction de biomolécules à une localisation donnée consistant à mettre en œuvre le complexe, tel que défini dans l'une des revendications 1 à 7 ou obtenu selon l'une des revendications 8 ou 9, dans un récipient ou sur un support.

11. Support ou récipient comprenant un complexe selon l'une des revendications 1 à 7 ou obtenu selon le procédé de la revendication 8, 9 ou 10 dans lequel le complexe se présente sous la forme d'un volume plein à l'état solide, dépourvu de volume intérieur.

12. Procédé d'extraction de biomolécules contenues dans un échantillon à tester comprenant :
- Une étape de solubilisation du complexe tel que défini dans les revendications 1 à 7, tel qu'obtenu selon la revendication 8 ou tel que contenu sur un support ou dans un récipient selon la revendication 9 ou 10, par la mise en contact dudit complexe avec un tampon aqueux, un tampon de lyse et/ou l'échantillon à tester lysé ou non lysé,
- Optionnellement si cette étape n'a pas encore eu lieu, une étape de mise en contact du complexe solubilisé avec l'échantillon à tester lysé ou non lysé,
- Optionnellement si l'échantillon à tester n'a pas déjà été lysé, une étape de lyse de l'échantillon à tester,
- Une étape de capture des biomolécules de l'échantillon à tester par les particules d'extraction libres de leurs mouvements dans de la matrice solubilisée, les biomolécules étant de préférence des acides nucléiques,
- Optionnellement, une étape de lavage des biomolécules capturées par les particules d'extraction,
- Optionnellement, une étape d'élution des biomolécules capturées par les particules d'extraction.

13. Procédé de détection de biomolécules contenus dans l'échantillon à tester, **caractérisé en ce qu'**il comprend en outre des étapes de la revendication précédente, les étapes suivantes:
- Optionnellement, une étape d'amplification des acides nucléiques lorsque les biomolécules extraites à l'issue du procédé selon la revendication 11 sont des acides nucléiques, de préférence l'amplification est réalisée par PCR,
- Une étape de détection des biomolécules par des méthodes appropriées.
